(19) Europäisches Patentamt
European Patent Office
Office européen des brevets

(11) **EP 1 906 811 B1**

(12) **EUROPÄISCHE PATENTSCHRIFT**

(45) Veröffentlichungstag und Bekanntmachung des Hinweises auf die Patenterteilung:
**20.04.2016   Patentblatt 2016/16**

(51) Int Cl.:
***A61B 3/12*** *(2006.01)*      ***A61B 3/14*** *(2006.01)*

(21) Anmeldenummer: **06762690.3**

(86) Internationale Anmeldenummer:
**PCT/EP2006/007089**

(22) Anmeldetag: **19.07.2006**

(87) Internationale Veröffentlichungsnummer:
**WO 2007/009761 (25.01.2007 Gazette 2007/04)**

(54) **EINRICHTUNG UND VERFAHREN ZUR BEOBACHTUNG, DOKUMENTATION UND/ODER DIAGNOSE DES AUGENHINTERGRUNDES**

DEVICE AND METHOD FOR MONITORING, DOCUMENTING AND/OR DIAGNOSING THE FUNDUS

DISPOSITIF ET PROCEDE POUR REALISER UN EXAMEN D'UN FOND D'OEIL ET ETABLIR, SUR LA BASE DE CE DERNIER, UNE DOCUMENTATION ET/OU UN DIAGNOSTIC

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **22.07.2005   DE 102005034332**

(43) Veröffentlichungstag der Anmeldung:
**09.04.2008   Patentblatt 2008/15**

(73) Patentinhaber: **Carl Zeiss Meditec AG
07745 Jena (DE)**

(72) Erfinder:
• **DICK, Manfred
07926 Gefell (DE)**
• **WESTPHAL, Peter
07743 Jena (DE)**

• **BUBLITZ, Daniel
07646 Rausdorf (DE)**
• **MOHR, Thomas
07745 Jena (DE)**

(74) Vertreter: **Beck, Bernard
Carl Zeiss Jena GmbH
Carl-Zeiss-Promenade 10
07745 Jena (DE)**

(56) Entgegenhaltungen:
**EP-A- 1 611 840        EP-A1- 0 631 757
EP-A1- 1 084 675        WO-A-92/02173
WO-A-02/080759        US-A- 5 836 872
US-A- 6 000 799        US-A1- 2004 064 057
US-A1- 2004 075 812        US-A1- 2004 105 074
US-A1- 2006 100 528        US-B1- 6 276 798
US-B1- 6 454 410        US-B1- 6 714 672**

**Beschreibung**

Die vorliegende Erfindung betrifft eine Einrichtung und ein Verfahren zur Beobachtung, Dokumentation und/oder Diagnose des Augenhintergrundes, wobei die Diagnose durch Auswertung der dokumentierten Abbilder des Augenhintergrundes, insbesondere den darin enthaltenen augendiagnostischen Merkmalen der Retina, wie beispielsweise die Geschwindigkeit des Blutflusses in den Adern des Augenhintergrundes erfolgt.

Nach dem bekannten Stand der Technik sind neben klassischen ophthalmologischen Geräten zur Untersuchung des Augenhintergrundes auch Laser-Scann-Systeme oder multispektrale Sensorsysteme mit optischer Strahlseparation im Beobachtungsstrahlengang bekannt. Die Systeme verfügen dabei meist über Mittel zur digitalen Bildaufnahme und -bearbeitung.

Bei den klassischen Funduskameras wirkt sich die Verwendung von mechanisch schwenkbaren Filtern zur Aufnahme von monochromatischen Aufnahmen, wie beispielsweise rot, grün, blau (RGB) nachteilig aus. Obwohl die Retina für Farbaufnahmen breitbandig mit weißem Licht, z. B. von Halogenlampen oder Xenon - Blitzlampen, beleuchtet wird, erfolgt die elektronische Auswertung nur in einem sehr schmalbandigen Bereich. Dazu werden Farbmasken auf den elektronischen Bildaufnahmesensor der Dokumentationskamera gelegt. Außerdem wird bei klassischen Funduskameras mit optischer Beobachtung die Retina kontinuierlich beleuchtet, so dass die Lichtbelastung für das zu untersuchende Auge besonders hoch ist. Die für die Diagnostik erforderliche schmalbandige Anregung im Bereich von 20 - 40 nm kann hierbei nur durch kostenintensive Filter realisiert werden, die zudem im Millisekunden-Bereich in und aus dem Strahlengang geschwenkt werden müssen. Ein Ophthalmoskop zur Beobachtung und Fotographie des Augenhintergrundes, welches für diese Gruppe charakteristisch ist, wird in der DE 25 12 427 A1 beschrieben.

Die US2004/0075812 beschreibt ein System zur Fundusbeobachtung mit einzelnen ansteuerbaren, monochromatischen Simulationslichtquellen.

Ein Augengerät mit einem optischen Bestrahlungssystem zum Bestrahlen eines Auges eines Patienten wird in der EP 1 114 608 B1 beschrieben. Das optische Bestrahlungssystem besteht hierbei aus einer Vielzahl von LEDs und einem optischen System zum Zusammenführen der optischen Lichtstrahlen der LEDs. Das Gerät verfügt weiterhin über eine Lichtmengensteuereinrichtung zum Verändern eines Verhältnisses der Lichtemissionsmengen der LEDs im Zusammenwirken mit dem Einführen und/oder dem Entfernen des Schutzfilters, das verhindert, dass der Behandlungslaserstrahl in das Auge eines Bedieners eintritt.

Die US 5,997,141 A offenbart ebenfalls ein Augengerät mit einem Bestrahlungssystem zum Bestrahlen eines Auges eines Patienten, welches eine Vielzahl von LEDs sowie eine Lichtmengensteuereinrichtung zum Steuern einer Bestrahlungslichtmenge von jeder der LEDs aufweist.

Eine programmierbare Lichtquelle wird in der US 3,760,174 A beschrieben. Auch hier kann durch einzeln gesteuerte LEDs gewährleistet werden, dass ein bestimmtes Bestrahlungsspektrum bereitgestellt wird. In der Regel handelt es sich bei dem bereitgestellten Bestrahlungsspektrum um ein gemischtes Licht-signal.

Bei der Verwendung von Laser-Scann-Systemen zur Untersuchung des Augenhintergrundes ist zum einen eine gleichzeitige Beobachtung nicht möglich und zum anderen sind die für konfokale Abbildungen notwendigen Laserquellen relativ teuer. Zudem muss der Laserstrahl zur Durchführung eines flächenhaften Scans der Retina über entsprechend aufwendige Einrichtungen abgelenkt werden.

Im Gegensatz dazu kann bei der Verwendung eines zweidimensionalen Multispektralspektrometers auf Einrichtungen zur Ablenkung verzichtet werden. Mit den in US 5,926,283 A1 und US 5,982,497 A1 beschriebenen Anordnungen können simultan spektral-getrennte, zweidimensionale Bilder des zu untersuchenden Objektes in Realzeit ermittelt werden.

Die bei multispektralen, z. B. "Quad-View"-Systemen vorgesehene optische Strahlseparation im Beobachtungsstrahlengang ist optisch nur sehr aufwendig realisierbar, was durch Probleme mit Reflexions- und Streulicht noch verstärkt wird. Außerdem sind hierbei nur geringe Bildwinkel von etwa 20° erreichbar. Auch hier ist die Lichtbelastung des zu untersuchenden Auges durch eine breitbandige Anregung mit weißem Licht sehr groß, außerdem gibt es Probleme mit Reflektionen und Streulicht im Beobachtungsstrahlengang.

Nach dem Stand der Technik werden Fundusaufnahmen mit Farbkamera aufgenommen, die eine Auflösung von 5-8 Megapixel aufweisen. Um die sehr hohen Lichtleistungen, die bei diesen unempfindlichen Kameras nötig sind zu erzeugen, werden Kombinationen von Halogenlampen (Beobachtung) und Blitzlampen (Dokumentation) eingesetzt.

Diese Systeme haben den Nachteil, dass sehr hohe Lichtleistungen eingesetzt werden müssen, um Fundusbilder mit der nötigen Dynamik zu erzeugen. Außerdem hat der Einsatz von Blitzlampen eine sehr komplizierte Steuerelektronik und erhöhten Serviceaufwand für den Ersatz ausgebrannter Blitzlampen zur Folge. Außerdem wird für viele Anwendungen, die mit der Funduskamera realisiert werden sollen die hohe Auflösung von 5-8 Megapixel nicht benötigt. Vielmehr werden oft empfindliche Sensoren benötigt, um die sehr schwachen Lichtsignale zu registrieren. Dies wird dadurch noch verstärkt, dass die bei den Aufnahmen nötige Lichtdosis weiter gesenkt werden soll, um die Lichtbelastung des zu untersuchenden Auges zu minimieren.

Der Blutfluss in der Netzhaut bzw. im Augenhintergrund hat eine wichtige physiologische Bedeutung für die Funktionsfähigkeit des Auges und ist möglicherweise auch ein Indikator für den sonstigen Gesundheitszustand einer Person. Die Mikrozirkulation der roten Blutkörperchen (Erythrozyten) wird zumindest in der Sepsis-Forschung und der Herzchirurgie als wichtiger physiologischer Parameter angesehen. Durch die Kombination mit Informationen über die Sauerstoff-Sättigung im gleichen Körperareal kann der Aussagewert noch wesentlich erhöht werden.

Es gibt bereits optische Verfahren (DE 30 41 178 C2), mit denen der gesamte Blutzufluss zum Augenhintergrund über den Dopplereffekt gemessen werden kann. Diese Messverfahren besitzen jedoch keine Ortsauflösung und sind daher nur von beschränktem Nutzen. Insbesondere kann damit auch keine Information über die Mikrozirkulation des Blutes gewonnen werden.

Die Ermittelung der Fliessgeschwindigkeit des Blutes in den Adern des Augenhintergrundes wurde bis jetzt z. B. über Doppler-Techniken untersucht (DE 30 41 178 C2). Dabei misst man an dem Eintrittspunkt der Adern in den Augenhintergrund die zur Augenhintergrundebene senkrechte Komponente der Blutflussgeschwindigkeit, indem man den Dopplerfrequenzshift einer im Blut zurückreflektierten Laserwelle bestimmt. Das Problem des Verfahrens besteht darin, das nur senkrechte Geschwindigkeitskomponenten im Bezug auf die Augenhintergrundsebene gemessen werden können. Das ist insofern ein Problem, als die Adern im Augenhintergrund tangential verlaufen und somit nur am Eintrittspunkt der Adern in den Augenhintergrund senkrechte Geschwindigkeitskomponenten auftreten. Daher wird eine Laserwelle aus zwei unterschiedlichen Richtungen in das Auge eingestrahlt, so das aus der Frequenzdifferenz der beiden Bündel auch die Tangentialkomponente der Blutflussgeschwindigkeit bestimmt werden kann. Außerdem ist die ortsaufgelöste Messung der Dopplerfrequenzverschiebung ein technisch aufwendiges Verfahren.

Die US 5,983,120 A1 beschreibt eine Lösung zur Reflexionsbild-Analyse, um eine non-invasive, in vivo Analyse von Gefäßsystemen durchzuführen. Dabei wird ein reflektiertes Bild in bezug auf den Hintergrund normalisiert und segmentiert, um einen interessierenden Bildausschnitt zu analysieren. Mit der vorgeschlagenen Lösung können beispielsweise die Konzentrationen von Hämoglobin, d. h. die Zahl roter Blutkörperchen pro Volumeneinheit Blut durch bis zu 0,5 mm dickes Gewebe ermittelt werden. Für die eine Messung des Blutflusses an der Netzhaut bzw. am Augenhintergrund ist die Lösung jedoch nicht ohne weiteres einsetzbar.

Ein Verfahren zum Sichtbarmachen einer ersten und einer zweiten Lage von Blutgefäßen wird in der EP 1 084 674 B1 beschrieben. Mit dem auf intravenös injizierten Fluoreszenzfarbstoffen basierenden Verfahren können zwar insbesondere die Choriokapillaris gegenüber den anderen Blutgefäßen in der Chorioidea sichtbar gemacht werden; eine ortsaufgelöste Blutflussmessung ist jedoch nicht möglich.

Auch die in der EP 0 801 534 B1 beschriebene Lösung zur optischen Darstellung des Blutkreislaufes und von abnormalen Gefäßstrukturen in der Aderhaut des Auges basiert auf dem intravenöses Injizieren eines Fluoreszenzfarbstoffs, wobei der Farbstoff die Gefäßanordnung (Vaskulatur) eines Auges füllt und durch Anregung Fluoreszenz ausstrahlt und dadurch die Gefäßanordnung auf einem angiographischen Bild abbildet.

Ein optisches Verfahren zur Blutflussmessung wird in WO 97/05538 A1 beschrieben. Als wesentliches Bauelement hat diese Lösung einen steuerbaren ortsaufgelösten Modulator, der über eine Anzahl von Bereichen Verfügt, die individuell in ihrer Transmission steuerbar sind. Jedem dieser Bereiche des Modulators ist ein strahlungsempfindlicher Bereich eines ortsauflösenden Detektors zugeordnet.

Die Schrift US 6,549,801 B1 beansprucht einen OCT-ähnlichen Aufbau. Das bedeutet, das als wesentliches Strukturmerkmal Licht des Primärarmes mit Referenzlicht zur Überlagerung und Interferenz gebracht wird.

Bei dem in JP10314118 A1 beschriebenen Verfahren wird die Fließgeschwindigkeit des Blutes gemessen, indem mit dem Punktdetektor ein zeitaufgelöstes Signal registriert wird. Durch Fouriertransformation dieses Signals erhält man die Cut-off-Frequenz als Maß für die Flussgeschwindigkeit. Dazu muss man aber das Signal des Detektors mit einer hohen Zeitauflösung registrieren. Diese Zeitauflösung kann mit einem ortsaufgelösten Detektor wie einer CCD-Kamera nur sehr schwer erreicht werden. Bei dem dargestellten Verfahren wird ein ortsaufgelöster Detektor ( zur einfachen 2d-Messung der relativen Fließgeschwindigkeit) mit einem schnellen Punktsensor (zur punktuellen Messung der absoluten Fließgeschwindigkeit) zu kombinieren. Das Verfahren ist dadurch technisch extrem anspruchsvoll und aufwendig.

Nach dem Stand der Technik gibt es ein Verfahren das Speckle-Korrelationstechnik genannt wird und eine sehr einfache und genaue Vermessung von Bewegungen streuender Flächen ermöglicht.

Beleuchtet man eine streuende Oberfläche mit einem Laser oder einer räumlich wie zeitlich sehr kohärenten Lichtquelle, so kann das von der Oberfläche zurückgestreute Licht im Raum interferieren und die typischen körnigen Strukturen (Speckle) erzeugen, die aus der Lasertechnik bekannt sind. Man sieht diese Speckle zum Beispiel wenn man einen sichtbaren Laser aufweitet und auf eine streuende Oberfläche richtet (Blatt Papier, Wand). Man unterscheidet zwischen subjektiven und objektiven Speckle. Subjektive Speckle entstehen, wenn man die streuende Oberfläche mit einer Optik auf den Kamerasensor abbildet. Die subjektiven Speckle sind genau so groß wie die optische Auflösung des abbildenden Systems. Das dann registrierte Streumuster bewegt sich mit der streuenden Oberfläche bei Verschiebungen mit und ermöglicht somit eine Bestimmung der Verschiebung.

Objektive Speckle entstehen, wenn der Kamerasensor ohne Optik in das von der Probe zurückgestreute Licht gestellt wird. Objektive Speckle reagieren auf Kippungen der Probenoberfläche. Die Größe der objektiven Speckle errechnet sich aus der Wellenlänge der Strahlung, dem Durchmesser der beleuchteten Probenfläche und dem Abstand zwischen Probenfläche und Kamerasensor.

Für die folgenden Ausführungen stellt der Begriff "Augenhintergrund" (oder lateinisch: Fundus) einen Sammelbegriff für folgende anatomischen Strukturen eines Auges dar:

- Netzhaut (Retina)
- Sehnervenkopf (Papille)
- Gefäße (Arteria und Vena centralis retinae)
- Gelber Fleck (Makula Lutea)
- Netzhautperipherie und Ora serrata (Übergang zwischen Netzhaut und dem Ziliarkörper).

Der Blutfluss beschreibt das Angebot an Nähr- und Wirkstoffen sowie an Abtransportkapazität für Stoffwechselprodukte, das einem Gefäßgebiet zur Verfügung gestellt wird. Stoffaustauschverhältnisse bestimmen aber darüber, ob das Angebot auch in erforderlicher Menge und Zeit in das zu versorgende Gewebegebiet gelangt.

Die Gefäßdurchmesser sind einerseits Stellglieder der lokalen Regulationsmechanismen und andererseits aber auch Angriffsort altersbedingter, pathologischer und therapeutischer Wirkungen. Daraus lässt sich ein hohes innovatives Potential der Gefäßanalyse für die Risikoanalyse, Früherkennung, Prognostik, Diagnostik und Therapie von Augenerkrankungen und ggf. auch für andere medizinische Fachdisziplinen ableiten.

Man muss davon ausgehen, dass das zeitliche und örtliche in vivo Verhalten des Durchmessers eines Gefäßsegmentes die Summenwirkung verschiedener miteinander konkurrierender, zueinander redundanter und einander ergänzender Regelketten widerspiegelt, die mit unterschiedlicher Dynamik und in den verschiedenen Gefäßbereichen auch mit unterschiedlicher Stärke zur Wirkung kommen.

Der vorliegenden Erfindung liegt die Aufgabe zugrunde, eine Lösung zur Aufnahme von empfindlichen als auch hochauflösenden Bildern der Retina zur Verfügung zu stellen, aus denen durch elektronische Nachbearbeitung und Auswertung Aussagen über mögliche Erkrankungen diagnostiziert werden können. Dabei soll die, vorzugsweise nonmydriatische Lösung Bildwinkel von bis zu 45° ermöglichen und die Lichtbelastung des zu untersuchenden Auges auf ein Minimum reduzieren.

Anhand von Bildern der Retina soll es möglich sein, den Blutfluss in der Netzhaut bzw. im Augenhintergrund zu bestimmen, um daraus wichtige Informationen über Funktionsstörungen und mögliche Erkrankungen frühzeitig diagnostizieren zu können.

Erfindungsgemäß wird die Aufgabe durch die Merkmale der unabhängigen Ansprüche gelöst. Bevorzugte Weiterbildungen und Ausgestaltungen sind Gegenstand der abhängigen Ansprüche.

Die erfindungsgemäße Lösung dient der Aufnahme von monochromatischen Bildern der Retina, beispielsweise rot, grün, blau oder auch infra-rot und der Aufnahme von Fluoreszenzbildern. Insbesondere lassen sich auch Fluoreszenzbilder im Infraroten Spektralbereich, unter Anwendung des Farbstoffes "I-docyaningreen" (kurz: ICG) aufnehmen.

Die Erfindung wird nachfolgend anhand eines Ausführungsbeispieles beschrieben. Dazu zeigen

Figur 1: eine Prinzipdarstellung einer Funduskamera zur Durchführung des erfindungsgemäßen Verfahrens,

Figur 2: ein ungünstig gewähltes Teilbild und das Ergebnis der resultierenden Korrelationsfunktion,

Figur 3: ein geeignetes Teilbild und das Ergebnis der resultierenden Korrelationsfunktion,

Figur 4: die erfindungsgemäße Anordnung zur Blutflussmessung am Augenhintergrund und

Figur 5: ein Diagramm zur Wellenlängenabhängigkeit der Absorptionsstärke des Hämoglobins.

Die erfindungsgemäße Einrichtung zur Beobachtung, Dokumentation und/oder Diagnose des Augenhintergrundes besteht aus einem ophthalmologischen Untersuchungsgerät, einem multispektralen, sequentiellen Beleuchtungsmodul, einem Bildaufnahmemodul, einem Steuer- und Sicherheitsmodul und einer Auswerteeinheit.

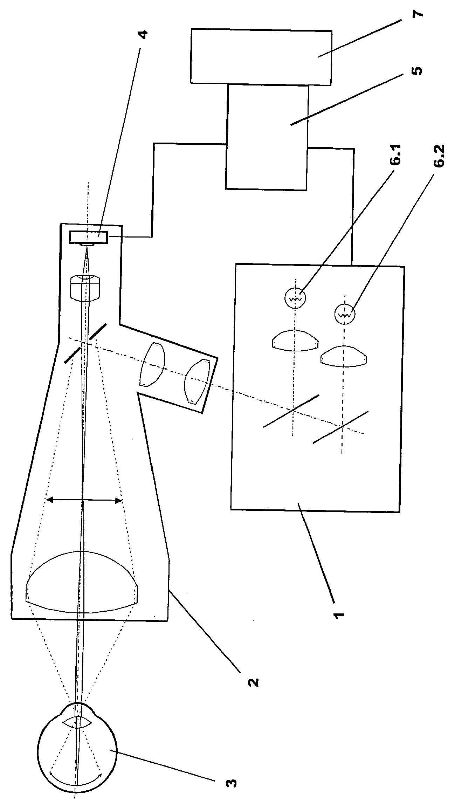

Eine Prinzipdarstellung einer Funduskamera zur Durchführung des erfindungsgemäßen Verfahrens ist in **Figur 1** dargestellt. Hierbei wird das vom multispektralen, sequentiellen Beleuchtungsmodul **1** ausgehende Licht über die Funduskamera **2** auf das Auge **3** abgebildet. Das vom Auge **3** reflektierte Licht wird von der Funduskamera **2** auf das Bildaufnahmemodul **4** abgebildet. Von einem Steuer- und Sicherheitsmodul **5** wird die zeitliche Abfolge, Dauer und Intensität der einzelnen Lichtquellen **6.1** und **6.2** des Beleuchtungsmoduls **1** gesteuert und die Lichtbelastung des Auges **3** überwacht. Die außerdem noch vorhandene Auswerteeinheit **7** regelt das Steuer- und Sicherheitsmodul **5** und verarbeitet die vom Bildaufnahmemodul **4** gelieferten Bilder hinsichtlich Kontrast, Bildschärfe, Farbfehler, Pixelfehler, Randabfall, Verzeichnungen, örtlichem Versatz u. ä. weiter und wertet diese auch aus. In einer besonderen Ausgestaltungsvariante ist zwischen dem Beleuchtungsmodul **1** und der Funduskamera **2** ein Lichtleiter bzw. ein Lichtleiterbündel zur Übertragung des Lichtes der einzelnen Lichtquellen **6.1** und **6.2** angeordnet, die mit Lichtmischstäben und/oder Mikrolinsen-Arrays gekoppelt sein können. Die Lichtmischstäbe und/oder Mikrolinsen-Arrays dienen dabei der Homogenisierung der Strahlung der Beleuchtung.

Das an das ophthalmologische Untersuchungsgerät angekoppelte Beleuchtungsmodul weist dabei mindestens zwei, in Intensität, Zeitpunkt und Dauer einzeln regelbare Einzellichtquellen auf, von denen monochromatisches Licht verschiedener Wellenlängen, vorzugsweise im Bereich von 350 - 900 nm abgestrahlt wird.

Vom ophthalmologischen Untersuchungsgerät wird das vom Beleuchtungsmodul kommende Licht über das zu untersuchende Auge auf dem Bildaufnahmemodul abgebildet, wobei das ebenfalls an das ophthalmologische Untersuchungsgerät angekoppelte Bildaufnahmemodul auf die Wellenlängen des vom Beleuchtungsmoduls emittierten Lichtes abgestimmt und mit deren Leuchtdauern synchronisiert ist.

Vom Steuer- und Sicherheitsmodul wird die zeitliche Abfolge, Dauer und Intensität der einzelnen Lichtquellen des Beleuchtungsmoduls gesteuert und die Lichtbelastung überwacht. Die Auswerteeinheit regelt das Steuer-und Sicherheitsmodul und bewertet, korrigiert, verbessert, kombiniert zur Auswertung die vom Bildaufnahmemodul übermittelten Aufnahmen des Fundus.

In einer vorteilhaften Ausgestaltung werden für das Beleuchtungsmodul Einzellichtquellen verwendet, deren Ein- und Ausschaltverzögerung unter 1 ms liegt. Das bedeutet, dass sich jede Strahlung innerhalb einer Zeit kleiner 1 ms von 0 auf 100% Intensität ein und auch innerhalb dieser Zeit von 100% auf 0% Intensität wieder ausschalten lässt.

Bei der Verwendung von mehr als zwei einzelnen Lichtquellen sind für deren Anordnung nicht nur zwei- sondern auch dreidimensionale Anordnungen denkbar. Die von den Einzellichtquellen erzeugten Strahlenbündel werden dabei über Spiegel und/oder Gitter, vorzugsweise mit dichroitischer Charakteristik, so miteinander gekoppelt, dass deren Strahlenbündel an der Koppelstelle zum ophthalmologischen Untersuchungsgerät in Apertur und Öffnungswinkel übereinstimmen und dort eine Abbildung mit möglichst konstantem Winkelspektrum erzeugen.

Die von den möglichst schmalbandigen Einzellichtquellen erzeugten Strahlenbündel stimmen an der Koppelstelle zum ophthalmologischen Untersuchungsgerät in Apertur und Öffnungswinkel überein und erzeugen eine Abbildung mit möglichst konstantem Winkelspektrum. Die Erfindung basiert auf dem Prinzip der geometrischen Teilung von Beleuchtungs- und Beobachtungsstrahlengang. Neben der in den meisten Funduskameras genutzten Ringpupillenbeleuchtung sind prinzipiell auch andere geometrischen Teilungen von Beleuchtungs- und Beobachtungsstrahlengang möglich.

Eine weitere Ausführung könnte eine kreisförmige Abbildung für den Beleuchtungsstrahlengang erzeugen. Der Beobachtungsstrahlengang ist dann ebenfalls kreisförmig und die beiden Strahlengänge liegen nebeneinander. Die optische Leistung der Einzellichtquellen sollte hierbei an der Schnittstelle mindestens 1 Milliwatt betragen.

Als Einzellichtquellen können hierbei insbesondere LEDs mit und ohne vorgeschaltete Filter oder auch Dioden-Laser verwendet werden, wobei eine möglichst gute Abdeckung des Farbraums erreicht werden sollte. In der Praxis orientiert man sich dabei an verfügbaren LEDs mit möglichst hoher Leistung. Im Einzelnen werden die Einzellichtquellen so ausgewählt, dass sowohl empfindliche und hochauflösende Bilder der Retina aufgenommen, als auch durch elektronische Nachbearbeitung und Auswertung Aussagen über mögliche Erkrankungen diagnostiziert werden können.

Beim Einsatz von Lasern zur Ausleuchtung des Augenhintergrundes bei sehr hohen Intensitäten wird in einer vorteilhaften Ausführung die Kohärenz der Laserstrahlung mittels einer geeigneten Einrichtung weitestgehend reduziert um eine homogene und flächige Ausleuchtung zu erzielen. In der Bewertung der Gefährdung durch optische Strahlung wird koheränte Strahlung als gefährlicher eingeschätzt als inkoheränte Strahlung; somit ist vorzugsweise stets inkoheränte Strahlung für die hochenergetische, flächige Ausleuchtung des Augenhintergrundes zu verwenden. Eine Einrichtung zur Reduzierung der Koheränz von Laserstrahlung kann zum Beispiel mittels eines Streufilters oder eines rotierenden Spiegels aufgebaut werden.

Für die Messung der Blutflussgeschwindigkeit über die Speckle-Korrelationstechnik nutzt man aber die kohärenten Eigenschaften des Laserlichtes aus. In diesem Fall darf die räumliche Kohärenz nicht durch zusätzliche Maßnahmen verschlechtert werden, was in diesem Ausführungsfall beispielsweise durch das Anhalten des rotierenden Siegels erreicht werden kann.

Ebenfalls kann für eine hochenergetische und flächige Puls-Beleuchtung des Augenhintergrundes eine Strahlungsquelle auf Basis einer Gasentladung verwendet werden; eine solche Strahlungsquelle emittiert inhomogene Strahlung. Ein Nachteil besteht jedoch darin, dass die Gasentladung nur relativ kurz (im Bereich weniger Millisekunden) betrieben werden kann und eine folgende Abkühlungsphase (im Bereich von 50 bis 100ms) erfordert.

Beispielsweise kann eine, infrarotes Licht abstrahlende LED als Monitor-LED zur Einstellung des Bildes verwendet werden. Blaues Licht abstrahlende LEDs werden für Fluoreszenzaufnahmen verwendet. Bei der Verwendung von 2 LEDs, die blaues (488nm) und grünes (532 oder 550nm) Lichtabstrahlen, kann die Makulapigmentdichte, d. h. die Xantophyll-Konzentration, bestimmt werden. Bei einer speziellen Methode der Fluoreszenz-Angiographie kann durch die Verwendung des Farbstoffes ICG, der im Bereich 730 - 780 nm angeregt wird, das Gefäßsystem der Aderhaut untersuchen werden.

LEDs mit den Farben Rot, Grün und Blau (RGB) sind beispielsweise für die Bestimmung eines resultierenden Farbbildes erforderlich. Bei dem sogenannten Quadview-Analogon werden vier LEDs mit speziellen gelben und grünen Wellenlängen verwendet. Dadurch ist es beispielsweise möglich einen skallierbaren Unterschied zwischen Sauerstoff-gesättigtem und ungesättigtem Blut zu detektieren.

Bei Verwendung von LEDs mit speziell abgestimmten Emissionswellenlängen ist durch elektronische Nachbearbeitung und Auswertung der monochromen Aufnahmen der Retina eine umfangreiche Diagnostik möglich. Die Anzahl der verwendeten LEDs ist dabei nicht begrenzt.

Vom Steuer- und Sicherheitsmodul werden insbesondere nur die Einzellichtquellen mit entsprechender Dauer und Intensität angesteuert, die für die jeweilige Untersuchung erforderlich sind. Das Steuer- und Sicherheitsmodul synchronisiert das Bildaufnahmemodul mit dem Beleuchtungsmodul, so dass zu jeder Wellenlänge eine, vorzugsweise auch mehrere zugehörige Bilder mit optimalem Kontrast, d. h. mit möglichst hohem Signal-Rausch-Verhältnis, realisiert werden. Mittels des Mikrolinsen-Arrays soll dabei der Füllfaktor der elektronischen Sensoren, der üblich im Bereich von 40 ... 60% liegt, deutlich auf >= 90% gesteigert werden. Als Bildaufnahmemodul wird beispielsweise eine empfindliche monochromatische Digitalkamera mit einer mittleren Auflösung von ca. 1 Megapixel verwendet. Der Bildwandelchip der Kamera kann dabei ein Mikrolinsenarray enthalten, um eine möglichst hohe Flächenfülldichte zu erreichen.

Die Auswerteeinheit verarbeitet die vom Bildaufnahmemodul gelieferten Bilder hinsichtlich Kontrast, Bildschärfe, Farbfehler, Pixelfehler, Randabfall, Verzeichnungen, örtlichem Versatz u. ä. weiter. Dabei werden mindestens ein aber vorzugsweise mehrere Bilder bei verschiedenfarbigen Beleuchtungszuständen mit einer Belichtungszeit von einigen Millisekunden aufgezeichnet.

Insbesondere werden von der Auswerteeinheit die vom Bildaufnahmemodul gelieferten Einzelbilder hinsichtlich deren Qualität bewertet, vorhandene Bildfehler, wie beispielsweise Bildschärfe, Pixelfehler, Randabfall und Verzeichnungen korrigiert, die Qualität der Aufnahmen hinsichtlich Kontrast und Schärfe verbessert und relevante Informationen für die funktionelle Diagnostik extrahiert.

Beispielsweise kann von der Auswerteeinheit aus den vom Bildaufnahmemodul gelieferten verschiedenen monochromatischen Bildern ein farbiges Bild des Fundus ermittelt werden. Es ist aber auch möglich, dass die Auswerteeinheit aus den vom Bildaufnahmemodul gelieferten Einzelbildern ein Bild mit einer Auflösung bis in den Sub-Pixelbereich ermittelt. Hierbei wird in beiden Fällen ein vorhandener örtlicher Versatz korrigiert, so dass die Einzelbilder exakt übereinander gelegt werden.

In einer weiteren vorteilhaften Ausgestaltung der erfindungsgemäßen Lösung ist das ophthalmologische Untersuchungsgerät eine Funduskamera, die vorzugsweise nach dem non-mydriatischen Prinzip arbeitet. Die Abbildung sollte dabei vorzugsweise ins Unendliche erfolgen und von der Augenlinse auf die Retina fokussiert werden.

Es ist vorgesehen, dass das ophthalmologische Untersuchungsgerät in einer weiteren Ausgestaltung über eine Einrichtung zur Überwachung und Kompensation der Augenbewegung verfügt. Dadurch kann die Augenbewegung Detektiert und der Sensor des Bildaufnahmemoduls 4 mit Hilfe eines Stellgliedes, beispielsweise eines Piezoantriebes, nachgeführt werden.

In einer weiteren vorteilhaften Ausgestaltung sind zur Messung des Blutflusses dem multispektralen, sequentiellen Beleuchtungsmodul **1** ein erstes Polarisationsfilter und dem Bildaufnahmemodul **4** ein zweites Polarisationsfilter. Diese Polarisationsfilter, die vorzugsweise als Linear- oder Zirkularpolarisatoren ausgeführt sind, befinden sich hierbei jeweils im kollimierten Beleuchtungs- bzw. Detektionsstrahlengang. Dabei werden die beiden Polarisationsfilter derart zueinander eingestellt, dass das direkt vom Auge reflektierte Licht maximal unterdrückt wird, so dass nur das mehrfach gestreute und damit depolarisierte Licht zum Bildaufnahmemodul 4 gelangt.

Hierbei wird der Augenhintergrund zur Messung des Blutflusses vom Beleuchtungsmodul **1** mit polarisiertem, vorzugsweise mit linear oder zirkular polarisiertem Licht beleuchtet, vom Bildaufnahmemodul **4** nur das vom Augenhintergrund reflektierte, mehrfach gestreute, depolarisierte Licht in Form von mindestens zwei unmittelbar aufeinanderfolgende Aufnahmen aufgenommen und für eine ortsaufgelöste Korrelationsanalyse an die Auswerteeinheit **7** weitergeleitet. Der Helligkeitskontrast auf dem Bildaufnahmemodul **4** ergibt sich dann unter anderem durch die Eindringtiefe des gestreuten Beleuchtungslichtes, da tiefer eingedrungenes Licht auf dem Rückweg zum Bildaufnahmemodul **4** häufiger gestreut und damit stärker depolarisiert wird. Typischerweise muss ein Photon mehr als zehnmal gestreut werden, um im statistischen Mittel depolarisiert zu sein.

Zur Kompensation der Doppelbrechung von Kornea, Augenlinse, Netzhaut und/oder optischen Bauelementen des ophthalmologischen Untersuchungsgerätes ist mindestens ein Doppelbrechungskompensator vorhanden, der vorzugsweise als variabler Doppelbrechungskompensator mit einer ortsabhängigen Kompensationscharakteristik ausgebildet ist.

Dabei kann es sich z. B. um einen nach dem Stand der Technik bekannten Babinet- oder einen Soleil-Kompensator handeln. Mit beiden Kompensatoren lassen sich unterschiedliche Stärken der Phasenverschiebung zw. s- und p-Licht sowie unterschiedliche Lagen der Polarisationsellipse kompensieren.

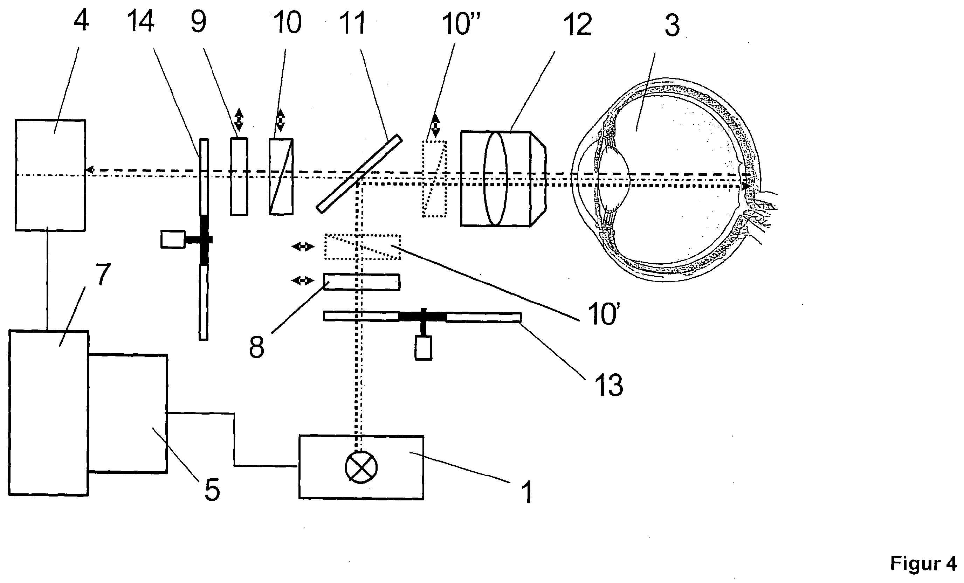

Eine erfindungsgemäße Anordnung zur Blutflussmessung am Augenhintergrund ist in **Figur 4** dargestellt. Ein erstes Polarisationsfilter **8** ist hierbei vor dem multispektralen, sequentiellen Beleuchtungsmodul **1,** ein zweites Polarisationsfilter **9** vor dem Bildaufnahmemodul **4** angeordnet.

Der zur Kompensation vorhandene Doppelbrechungskompensator **10** wird vorzugsweise im Detektionsstrahlengang vor dem Polarisationsfilter **9** angeordnet.

Grundsätzlich kann der Doppelbrechungskompensator **10'** aber auch im Beleuchtungsstrahlengang, zur Vorkompensation der Doppelbrechung, oder zwischen Strahlteiler **11** und Objektiv **12** (gekennzeichnet als **10"**) angeordnet sein. Prinzipiell ist es sogar möglich mehrere Doppelbrechungskompensatoren **10, 10'** und **10"** zu verwenden.

Da die Kornea-Doppelbrechung zwar im zentralen Bereich (nahe der optischen Achse) relativ konstant ist, jedoch weiter außerhalb räumlich variieren kann, ist es vorteilhaft Doppelbrechungskompensator **10** zu verwenden, die eine räumlich variierende Kompensationscharakteristik aufweisen.

In jedem Fall werden alle die Polarisation beeinflussenden Elemente, wie Polarisationsfilter **8, 9** und Doppelbrechungskompensator **10** so zueinander eingestellt, dass der Inhalte der Blutgefäße mit maximalem Kontrast auf dem bildgebenden Sensor erscheint.

Für die Bildgebung wird hierbei eine laterale Auflösung von 1 bis 7 $\mu$m verwendet, um die roten Blutkörperchen (Erythrozyten), die beim Menschen einen Durchmesser von ca. 7,5 $\mu$m aufweisen, zumindest grob auflösen zu können. Auflösungen von 7 bis 20 $\mu$m könnten jedoch auch noch ausreichen, um einen hinreichenden räumlichen Kontrast aus dem Inneren der Blutgefäße zu erhalten.

Zur Messung des Blutflusses bzw. der Blutflussgeschwindigkeit werden mindestens zwei zeitlich unmittelbar aufeinanderfolgende Aufnahmen des Augenhintergrundes einer ortsaufgelösten Korrelationsanalyse unterzogen. Dabei wird bestimmt, wie weit sich in der Zeit zwischen den Aufnahmen die Helligkeitsprofile längs einzelner Blutgefäße verschoben haben. Diese Helligkeitsprofile werden durch die zufällige räumliche Verteilung der Erythrozyten verursacht, die in jedem Blutgefäß trotz des Blutflusses eine gewisse Zeit erhalten bleibt.

In einer weiteren vorteilhaften Ausgestaltung sind zur Messung des Sauerstoffgehaltes des Blutes wird dem multispektralen, sequentiellen Beleuchtungsmodul **1** oder dem Bildaufnahmemodul **4** mindestens ein Farbfilter vorgeordnet und vom Bildaufnahmemodul **4** jeweils Aufnahmen des Augenhintergrundes mit bzw. ohne Farbfilter aufgenommen und an die Auswerteeinheit **7** weitergeleitet. Auf die Verwendung von Farbfiltern kann hierbei verzichtet werden, wenn vom multispektralen, sequentiellen Beleuchtungsmodul **1** Licht der entsprechenden Wellenlängen zur Verfügung gestellt werden kann.

Bei der Messung des Sauerstoffgehaltes des Blutes geht es stets um eine ortsaufgelöste Messung der Sauerstoffsättigung, also um eine Kartierung der Sauerstoffsättigung über den Fundus, wobei zwischen der Sauerstoffsättigung in den Gefäßen und der Sauerstoffsättigung im Kapillargebiet unterschieden werden muss.

Zur Messung der Sauerstoffsättigung müssen mindestens zwei Aufnahmen bei 2 verschiedenen Beleuchtungsspektren erfolgen. Die vom Beleuchtungsspektrum abhängige (ortsabhängige) Reflektivität des Fundus ist unter anderem durch das Absorptionsspektrum von oxigeniertem und deoxigeniertem Hämoglobin bestimmt. Daneben ist es möglich dass das rückreflektierte Spektrum gleichzeitig von weiteren zum Zeitpunkt der Messung unbekannten Parametern wie z. B. der Gefäßdicke oder dem Hämatokrit (Volumenanteil der roten Blutkörperchen am gesamten Blut) abhängt. Durch Beleuchtung und Detektion in geeigneten Wellenlängenbereichen ist es möglich all diese Parameter gemeinsam aus den gemessenen Daten zu extrahieren (ebenfalls ortsaufgelöst). Im Fall mehrerer (>2) zum Zeitpunkt der Messung unbekannter Parameter (Anzahl: n) müssen mindestens n Messungen mit jeweils paarweise verschieden, für das konkrete Problem geeigneten Beleuchtungsspektren durchgeführt werden.

Zur Messung des Sauerstoffgehaltes des Blutes ist es erforderlich, dass vom Bildaufnahmemodul **4** jeweils Aufnahmen des Augenhintergrundes bei zwei verschiedenen Wellenlängen aufgenommen und an die Auswerteeinheit **7** weitergeleitet werden. Dazu wird mindestens ein, vorzugsweise jedoch zwei Farbfilter verwendet, die abwechselnd vor dem Beleuchtungsmodul **1** oder dem Bildaufnahmemodul **4** angeordnet sind und für eine Aufnahme in den Strahlengang eingebracht werden. Zum Wechseln der Beleuchtungs- bzw. Detektionswellenlänge sind hierbei zwei Filterräder vorhanden.

Aufnahmen des Augenhintergrundes bei zwei verschiedenen Wellenlängen können hierbei derart erfolgen, dass die Wellenlänge des Beleuchtungslichtes verändert wird, oder dass bei einer breitbandigen Beleuchtung Farbfilter vor die Bildaufnahmemodul **4** gebracht werden.

Der **Figur 4** ist zu entnehmen, dass die Farbfilter dabei vorteilhaft auf einem Filterrad angeordnet sein können. Durch geeignete Kombination von Farbfiltern in Filterrad **13** und Filterrad **14** können mit dieser Anordnung zusätzlich Fluoreszenzaufnahmen vom Augenhintergrund gemacht werden.

Um konventionelle Funduskamera-Aufnahmen machen zu können, sind alle Polarisationsfilter und Kompensatoren so gestaltet, dass diese aus den Strahlengängen ausgeschwenkt werden können.

Für diesen Zweck weisen die Filterwechsler Positionen ohne Filter (bei Verwendung von Farbkameras) bzw. Positionen mit Rot-, Grün- und Blau-Filtern auf (bei Verwendung von Schwarz-Weiß-Kameras) auf. Auch hier kann auf die Verwendung von Farbfiltern verzichtet werden, wenn vom multispektralen, sequentiellen Beleuchtungsmodul **1** Licht der entsprechenden Wellenlängen zur Verfügung gestellt werden kann.

Vorteilhaft ist es hierbei, wenn die erforderlich Aufnahmen des Augenhintergrundes bei Wellenlängen von ca. 650 nm und ca. 810 nm aufgenommen werden. Während die Spektren von oxigeniertem und deoxigeniertem Hämoglobin bei 810 nm keinen Unterschied aufweisen, unterscheiden sich diese bei 650 nm sehr stark, so dass die Sauerstoffsättigung direkt ermittelt werden kann. Bei einer Wellenlänge von 650 nm absorbiert der rote Blutfarbstoff (Hämoglobin) besonders stark, so dass die Blutgefäße bzw. deren Inhalt dunkler als das umgebende Gewebe dargestellt wird. Dadurch kann gleichzeitig die lokal unterschiedliche Absorption des Augenhintergrundes kompensiert werden. Dazu werden entweder ein Filter verwendet, welches für eine der Wellenlänge durchlässig ist, während die andere Wellenlänge vom Beleuchtungsmodul **1** abgedeckt ist, oder es werden zwei Filter verwendet, deren Durchlassbereich diesen beiden Wellenlängen entspricht.

Hierbei ist zu berücksichtigen, dass die Absorption von Hämoglobin unterhalb von ca. 610 nm besonders stark ist und die Absorption von oxygeniertem Hämoglobin im Bereich um 690 nm ein Minimum aufweist. Wenn es um einen möglichst guten Kontrast vor dem Augenhintergrund geht, müssen Arterien (stark oxigeniertes Hämoglobin) auf alle Fälle im Bereich < 610 nm und Venen (weniger stark oxigeniertes Hämoglobin) unterhalb von ca. 800 nm abgebildet werden. Für einen guten Kontrast für beide Gefäßtypen mit kleinem Durchmesser erreichen, ist eine Wellenlänge unterhalb von ca. 600 nm vorzusehen. In diesem Wellenlängenbereich werden die Blutgefäße in der Regel dunkler dargestellt als das umgebende Gewebe.

Ein weiterer wichtiger Einflussfaktor auf den Kontrast ist die lokal unterschiedliche Absorption des Augenhintergrunds. Um diesen Effekt zu maximieren, verwendet man vorzugsweise Beleuchtungswellenlängen, bei denen der rote Blutfarbstoff (Hämoglobin) stark absorbiert (ca. 500...600 nm). Dadurch erscheinen die Blutgefäße bzw. deren Inhalt dunkler als das umgebende Gewebe.

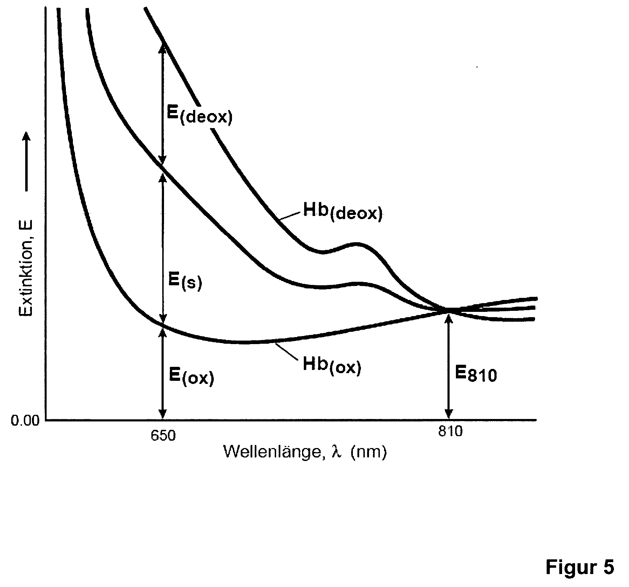

Zur Messung des Sauerstoffgehaltes des Blutes bzw. dessen Sauerstoffsättigung wird ebenfalls ortsaufgelöst bestimmt, indem vom Bildaufnahmemodul **4** Aufnahmen unter Verwendung von wenigstens zwei verschiedenen Wellenlängen gemacht werden. Dabei wird ausgenutzt, dass sich die Absorptionsspektren von sauerstoffreichem ($HB_{ox}$) und sauerstoffarmem Blut ($HB_{deox}$) deutlich unterscheiden. Dazu zeigt **Figur 5** ein Diagramm zur Wellenlängenabhängigkeit der Absorptionsstärke des Hämoglobins. Bevorzugte Wellenlängen liegen bei ca. 650 nm und ca. 810 nm.

Mit der vorgeschlagenen technischen Lösung wird eine Einrichtung und ein Verfahren zur Beobachtung, Dokumentation und/oder Diagnose des Augenhintergrundes zur Verfügung gestellt, mit der der Blutfluss, oder genauer die Fliessgeschwindigkeit des Blutes, an der Netzhaut bzw. am Augenhintergrund ortsaufgelöst gemessen werden kann. Idealerweise ist die Lösung mit kleinen, reversiblen Modifikationen auch geeignet, den Sauerstoffgehalt des Blutes zu messen, wobei die Gesamtlösung leicht in eine konventionelle Funduskamera integrierbar ist.

Bei dem erfindungsgemäßen Verfahren zur Beobachtung, Dokumentation und/oder Diagnose des Augenhintergrundes wird mit Hilfe eines ophthalmologischen Untersuchungsgerätes das Licht eines multispektralen, sequentiellen Beleuchtungsmoduls über das Auge auf ein Bildaufnahmemodul abgebildet. Das an das ophthalmologische Untersuchungsgerät angekoppelte, aus mindestens zwei, in Intensität und Dauer einzeln regelbare Einzellichtquellen bestehende Beleuchtungsmodul strahlt dazu monochromatisches Licht verschiedener Wellenlängen, vorzugsweise im Bereich von 350 - 900 nm ab, die vom ophthalmologischen Untersuchungsgerät über das zu untersuchende Auge auf das ebenfalls an das ophthalmologische Untersuchungsgerät angekoppelte Bildaufnahmemodul, welches auf die Wellenlängen des vom multispektralen Beleuchtungsmoduls emittierten Lichtes abgestimmt und mit deren Leuchtdauer synchronisiert ist, abgebildet wird. Vom vorhandenen Steuer- und Sicherheitsmodul wird die zeitliche Abfolge, Dauer und Intensität der Einzellichtquellen des Beleuchtungsmoduls gesteuert und die Lichtbelastung des zu untersuchenden Auges überwacht. Die Auswerteeinheit regelt das Steuer- und Sicherheitsmodul und bewertet, korrigiert, verbessert, kombiniert zur Auswertung die vom Bildaufnahmemodul übermittelten Aufnahmen des Fundus.

In einer vorteilhaften Ausgestaltung werden für das Beleuchtungsmodul Einzellichtquellen verwendet, deren Ein- und Ausschaltverzögerung unter 1 ms liegt. Das bedeutet, dass sich jede Strahlung innerhalb einer Zeit kleiner 1 ms von 0 auf 100% Intensität ein und auch innerhalb dieser Zeit von 100% auf 0% Intensität wieder ausschalten lässt.

Die von den möglichst schmalbandigen Einzellichtquellen erzeugten Strahlenbündel stimmen an der Koppelstelle zum ophthalmologischen Untersuchungsgerät in Apertur und Öffnungswinkel überein und erzeugen vorzugsweise eine kreisförmige oder kreisringförmige Abbildung mit möglichst konstantem Winkelspektrum. Die optische Leistung der Einzellichtquellen sollte hierbei an der Schnittstelle mindestens 1 Milliwatt betragen.

Vom Steuer- und Sicherheitsmodul werden insbesondere nur die Einzellichtquellen mit entsprechender Dauer und Intensität angesteuert, die für die jeweilige Untersuchung erforderlich sind. Das Steuer- und Sicherheitsmodul synchronisiert das Bildaufnahmemodul mit dem Beleuchtungsmodul, so dass zu jeder Wellenlänge eine, vorzugsweise aber auch mehrere zugehörige Bilder mit optimalem Kontrast, d. h. mit möglichst hohem Signal-Rausch-Verhältnis, realisiert werden. Als Bildaufnahmemodul wird beispielsweise eine empfindliche Monochromkamera mit mittlerer Auflösung von ca. 1 Megapixel verwendet. Der Bildwandelchip der Kamera kann dabei ein Mikrolinsenarray enthalten, um eine möglichst hohe Flächenfülldichte zu erreichen.

Die zeitliche Reihenfolge der einzelnen Spektralfarben ist vorteilhaft so zu wählen, dass zeitlich zuerst die Wellenlänge emittiert wird, die die geringste Reizung des Auges, insbesondere des Pupillenreflexes, verursacht. Vorteilhaft sollte daher zunächst eine Aufnahme ohne eine Reizung im infraroten Bereich, gefolgt von einer Aufnahme im roten Bereich, bei einer geringen Reizung erfolgen. Nachdem eine Aufnahme im blauen Bereich, bei relativ geringe Reizung erfolgte, wird abschließend eine Aufnahme im grünen Bereich gemacht, bei der das Auge am empfindlichsten ist und eine maximale Reizung erfolgt.

Weiterhin ist für die Aufnahme von farbigen Fundusbildern die Intensität der einzelnen Spektralfarben vorteilhaft an die Verteilung der Farbinformation der Retina anzupassen. Die Retina weist vor allem Rot-Anteile auf (ca. 60 ... 80%), gefolgt von Anteilen im grünen Bereich (20 ... 30%). Der Anteil von blauen Farbinformationen ist am geringsten (5 ... 15%). Vorteilhaft sollte daher die multispektrale Beleuchtung einen relativ hohen Rot-Anteil aufweisen.

Wie bereits beschrieben können als Einzellichtquellen hierbei insbesondere LEDs mit und ohne vorgeschaltete Filter oder auch Dioden-Laser verwendet werden, wobei die Einzellichtquellen so ausgewählt, dass sowohl empfindliche und hochauflösende Bilder der Retina aufgenommen, als auch durch elektronische Nachbearbeitung und Auswertung Aussagen über mögliche Erkrankungen diagnostiziert werden können.

Die Auswerteeinheit verarbeitet die vom Bildaufnahmemodul gelieferten Bilder hinsichtlich Kontrast, Bildschärfe, Farbfehler, Pixelfehler, Randabfall, Verzeichnungen, örtlichen Versatz u. ä. weiter. Dabei werden mindestens ein aber vorzugsweise mehrere Bilder bei verschiedenfarbigen Beleuchtungszuständen mit einer Belichtungszeit von einigen Millisekunden aufgezeichnet.

Insbesondere werden von der Auswerteeinheit die vom Bildaufnahmemodul gelieferten Einzelbilder hinsichtlich deren Qualität bewertet, vorhandene Bildfehler, wie beispielsweise Bildschärfe, Pixelfehler, Randabfall und Verzeichnungen korrigiert, die Qualität der Aufnahmen hinsichtlich Kontrast und Schärfe verbessert und relevante Informationen für die funktionelle Diagnostik extrahiert.

So könne beispielsweise Kontrastunterschiede ausgeglichen werden, indem vom Bildaufnahmemodul **4** statt einer monochrome Aufnahme bei Beleuchtung des Augenhintergrundes mit Weißlicht drei monochrome Aufnahmen bei Beleuchtung mit blauem, grünen und roten Licht und die Auswerteeinheit **7** aus diesen drei Aufnahmen eine Gesamtaufnahme ermittelt.

In einer Ausgestaltung des Verfahrens wird der Augenhintergrund vom multispektralen, sequentiellen Beleuchtungsmodul **1** beleuchtet und vom Bildaufnahmemodul **4** mindestens zwei unmittelbar aufeinanderfolgende Aufnahmen mit unterschiedlicher Belichtungszeit aufgenommen, die für eine ortsaufgelöste Kombination und Auswertung an die Auswerteeinheit **7** weitergeleitet werden. Im einzelnen wird dabei vom Bildaufnahmemodul **4** eine erste Aufnahme des Augenhintergrundes mit normaler Belichtungszeit und eine unmittelbar aufeinanderfolgende zweite Aufnahme bei Überbelichtung aufgenommen, wobei von der Auswerteeinheit **7** aus der überbelichteten Abbildung die überstrahlten Bereiche ausgeschnitten und durch die entsprechenden Bereiche der normalbelichteten Aufnahme ersetzt werden, um so eine dynamischere Aufnahme zu erhalten.

Bei einer weiteren vorteilhaften Ausgestaltung zur Messung des Blutflusses am Augenhintergrund strahlt mindestens eine Einzellichtquelle des multispektralen, sequentiellen Beleuchtungsmoduls **1** räumlich und zeitlich sehr kohärentes Licht ab, wozu für diese Einzellichtquelle ein Laser verwendet wird, der vorzugsweise gepulst betrieben wird.

Hierbei wird der Augenhintergrund zur Messung des Blutflusses vom multispektralen, sequentiellen Beleuchtungsmodul **1** mit räumlich und zeitlich sehr kohärentem Licht beleuchtet, vom Bildaufnahmemodul **4** in Form von mindestens zwei Aufnahmen hoher zeitlicher Auflösung aufgenommen und für eine ortsaufgelöste Korrelationsanalyse zur Bestimmung von Strecke und Richtung des Blutflusses an die Auswerteeinheit **7** weitergeleitet wird, zur Bestimmung der Geschwindigkeit des Blutflusses werden mindestens zwei weitere Aufnahmen hoher zeitlicher Auflösung bei räumlich und zeitlich sehr kohärenter Beleuchtung des Augenhintergrundes aufgenommen und für eine ortsaufgelöste Korrelationsanalyse an die Auswerteeinheit **7** weitergeleitet. Das Bildaufnahmemodul **4** wird dabei vom Steuer- und Sicherheitsmodul **5** synchron zum vorzugsweise gepulst betriebenen Beleuchtungsmodul **1** gesteuert.

In einer besonders vorteilhaften Ausgestaltung wird für das Bildaufnahmemodul **4** eine handelsübliche Digitalkamera verwendet. Bei einer handelsüblichen Digitalkamera mit einer Auflösung beispielsweise von 1Mpixel dauert das Auslesen und Speichern des auf dem Chips dargestellten Bildes zirka 60 ms. Dies zeitliche Auflösung für die Registrierung der schnellen Specklebewegungen jedoch nicht ausreichend. Um dabei die hohe Zeitauflösung zu erreichen, wird folgendes Beleuchtungs- und Bildaufnahmeverfahren angewendet.

Das gepulst betriebene Beleuchtungsmodul **1** wird für die Doppelaufnahmen hoher zeitlicher Auflösung bei räumlich und zeitlich sehr kohärenter Beleuchtung so vom Steuer- und Sicherheitsmodul **5** gesteuert, dass ein erster Beleuchtungsblitz am Ende der Belichtungszeit für die erste Aufnahme und ein zweiter Beleuchtungsblitz am Anfang der Belichtungszeit für die zweite Aufnahme ausgelöst wird. Dadurch wird die zeitliche Auflösung für dieses Doppelbild im wesentlichen nur durch die Blitzzeit des Lasers begrenzt und kann so praktisch auf wenige Mikrosekunden reduziert werden.

In einem ersten Verfahrensschritt werden die beiden, vom Bildaufnahmemodul **4** mit hoher zeitlicher Auflösung aufgenommenen Abbilder des Augenhintergrundes für eine ortsaufgelöste Korrelationsanalyse zur Bestimmung von Strecke und Richtung des Blutflusses an die Auswerteeinheit **7** weitergeleitet. Dabei zeigt die Korrelation der beiden Bilder gegeneinander die Verschiebung des Auenhintergrundes während der Messung in Strecke und Richtung. Werden die Bilder voneinander abgezogen, so erkennt man eine (verspeckelte) gleichmäßig graue Fläche mit sich abzeichnenden helleren Adern. Der Grund für die hellen Adern liegt in dem Blutfluss innerhalb der Adern begründet.

Um jedoch aus der Helligkeitsdifferenz der Adern zum Augenhintergrund auf die Blutgeschwindigkeit schließen zu können, ist zu bestimmen, wie viel Licht aus der Wand der Ader und wie viel Licht aus dem Blutstrom innerhalb der Adern zurückgestreut wird. Dazu werden mindestens zwei weitere Aufnahmen hoher zeitlicher Auflösung bei räumlich und zeitlich sehr kohärenter Beleuchtung des Augenhintergrundes aufgenommen und für eine ortsaufgelöste Korrelationsanalyse an die Auswerteeinheit **7** weitergeleitet.

Diese beiden Doppelbilder werden gegeneinander korreliert, danach voneinander abgezogen und anschließend dessen Betrag gebildet, wodurch die Helligkeitsdifferenz der Adern in bezug auf den Augenhintergrund gemessen. Aus diesem Wert als Funktion der beiden Zeitabstände der Doppelbilder kann dann bestimmt werden, wie viel Licht aus dem Blutfluss zurückgestreut wird und mit welcher Geschwindigkeit das Blut absolut in den Adern fließt. Um die Zeitauflösung mit möglichst wenigen Bildern zu erreichen, ist es auch möglich wie beschrieben Doppelbilder mit sehr kurzer zeitlicher Folge aufzunehmen und gleichzeitig die Impulsdauer der beiden Einzelimpulse zu variieren. In diesem Fall kann dann die Blutflussgeschwindigkeit auch aus den Eigenschaften der Autokorrelationsfunktion von Teilabschnitten der Fundusbilder bestimmt werden.

Die Bilder der Funduskamera zeigen durch eine kreisförmige Maske den Fundus des zu untersuchenden Auges. Es werden Bilder bzw. Filmsequenzen von mehreren Bildern des Augenhintergrundes aufgezeichnet und ausgewertet. Da alle Bilder der Sequenz mit dem ersten Bild verglichen werden, muss darauf geachtet werden, dass das erste Bild möglichst scharf ist und keine Bewegungsunschärfen oder Interlaceartefakte enthält. Es wird in jedem Bild ein quadratisches Teilbild mit $2^n \times 2^n$ Pixel (n=6 ... 8) aus der mittleren Bildregion ausgewählt. Das Teilbild wird für jeden Film so gewählt, dass möglichst viele scharfe Strukturen in dem Teilbild enthalten sind, weil dadurch die Genauigkeit der Korrelation verbessert wird. Die Teilbilder werden mit TB1...TBn bezeichnet. Die Kreuzkorrelationsfunktionen der Bilder der Sequenz gegen das erste Bild der Sequenz werden mit K(TBn; TB1) bezeichnet. Die Kreuzkorrelationsfunktion ergibt sich dann nach:

$$F\big[K(TBn \ ; \ TB1)\big] = F[TBn] \cdot F[TB1]^*$$

$$K(TBn \ ; \ TB1) = F^{-1}\big[F[TBn] \cdot F[TB1]^*\big]$$

wobei

F der Operator für die Fouriertransformation und
$F^{-1}$ der Operator für die Rücktransformation ist und
* komplex konjugiert bedeutet.

Die Größe der Teilbilder muss optimiert werden, um die Geschwindigkeit und Genauigkeit des Verfahrens zu optimieren. Die Geschwindigkeit für die Berechnung der schnellen 2-dimensionalen FFT eines m x m Pixel großen Bildes ist proportional zu:

$$m^2 \cdot \ln(m)$$

Im Ergebnis der 2-dimensionale FFT, bei der erst von jeder Zeile und dann von jeder Spalte die FFT berechnet wird, entsteht ein 2-dimensionales Bild im Fourierraum. Die Größe m der Teilbilder muss so gewählt werden, dass sie erstens deutlich größer als die maximal zu erwartende Verschiebung der Bilder ist und zweitens eine Mindestgröße nicht unterschreitet, damit sich die durch das Pixelrauschen der Kamerabilder verursachten Fehler in der Korrelationsfunktion über die Teilbilder herausmitteln. Für die typischen Bilder vom Augenhintergrund erweisen sich m=64, 128 und 256 Pixel als sinnvoll.

Als nächstes wird in der Kreuzkorrelationsfunktion der Pixel mit der maximalen Helligkeit bestimmt. Ist der Pixel mit den Koordinaten (0,0) der Hellste, so sind die beiden Bilder nicht gegeneinander verschoben. Ist ein anderer Pixel in der Korrelationsfunktion der hellste, so gibt der Abstand und die Richtung zum Koordinatenursprung die Stärke und Richtung der Verschiebung an (Korrelationsbilder werden im Ursprung periodisch in x- und y-Richtungen fortgesetzt). Die Genauigkeit der Bestimmung der Verschiebung beträgt maximal ein Pixel.

Von der Auswerteeinheit können die um den bestimmten Wert der Verschiebung korrigierten Bilder überlagert und farbige bzw. gemittelte monochromatische Bilder ermittelt werden.

Das Grundprinzip der Bildmittelung besteht darin, die Bilder gegeneinander zu korrelieren um die Bildverschiebung zwischen den Aufnahmen zu bestimmen. Mit Hilfe dieser Daten können mehrere Bilder pixelrichtig überlagert und gemittelt werden. Dadurch erhält man Bilder mit geringerem Pixelrauschen d. h. besserem Bildkontrast.

Im Einzelnen kann der bestimmte Wert der Verschiebung dazu genutzt werden mehrere, bei einer Farbe aufgenommene Einzelbilder ohne Fehler zu überlagern und zu mitteln, um Bilder mit einem höheren Kontrast, d. h. einem geringeren Rauschen, also mit einer höheren Dynamik zu erhalten.

Es ist aber auch möglich, den bestimmten Wert der Verschiebung dazu zu nutzen, bei unterschiedlichen Farben aufgenommene Einzelbilder (Farbteilbilder) ohne Fehler zu überlagern und so ein Farbbild des Fundus zu bestimmen. Dies hat den Vorteil, dass farbige LEDs mit sehr hoher spektraler Intensität eingesetzt werden können. Die Farbinformationen des Fundusbildes erhält man bei dieser Art der Messung durch die farbige Beleuchtung, anstatt durch eine Farbmaske vor dem Empfängerchip.

Es könnten zum Beispiel bei der Live-Fundusbeobachtung alle Teilbilder im Videotakt gemessen, gespeichert und anschließend überlagert werden. Dadurch währe gar keine gepulste Aufnahme mit Blitzlichtquelle mehr notwendig und man würde trotzdem Bilder mit vergleichbarer oder höhere Dynamik erhalten.

Es zeigt sich, dass dieses Verfahren Bilder mit einer mindestens 10-fach größeren Helligkeit erzeugt, als bei der Verwendung einer Weißlichtquelle mit RGB-Kamera.

Eine weitere Modifikation des Verfahrensschrittes bestände darin, das die Farbteilbilder jeweils über unterschiedliche Bildzahlen gemittelt würden. Der Grund dafür liegt in den spektralen Eigenschaften des Fundus. Ein Farbbild vom Fundus trägt den Hauptteil der Informationen im roten Teilbild (weil der Augenhintergrund rötlich ist). Deshalb würde man sehr viel mehr Teilbilder im roten Farbbereich mittel, als von den anderen Farbteilbildern. Nach der Überlagerung würde das Bild rauschärmer erscheinen und man erhält so das beste Signal/Rausch-Verhältniss in der kürzesten Messzeit.

Der wesentlichste Vorteil des Verfahrens besteht in der Möglichkeit die Auflösung der Bilder erheblich zu vergrößern.

Dazu wird ein Verfahren verwendet, das zum Stand der Technik gezählt werden kann, das aber in einem Punkt wesentlich abgeändert wird. Dieses Verfahren soll jetzt kurz beschrieben werden.

Als erstes nimmt man $n^2$ Bilder auf, die gegeneinander jeweils in der x- und unabhängig in der y-Richtung um den n-ten Teil eines Pixels verschoben sind. Die $n^2$ vom Bildaufnahmemodul gelieferten und um jeweils den n-ten Teil eines Pixel in x- und y-Richtung verschoben aufgenommenen Bilder werden von der Auswerteeinheit in ein n-mal so großes Bildfeld pixelweise eingeschrieben, dass jeweils n Spalten und n Zeilen für die anderen Bilder frei bleiben, d. h. die $n^2$ Bilder werden ineinander geschachtelt. Das in jeder Richtung n-mal so große, volle Bildfeld wird danach fouriertransformiert und durch die Fouriertransformation der Verteilung eines Korrekturbildes geteilt. Anschließend wird dieses Bild durch eine inverse Fouriertransformation zurücktransformiert und so ein Bild mit n-facher Auflösung bestimmt. Die Korrektur im Fourierraum wird nötig, da die ineinander geschachtelten Bilder mit relativ zu großen Pixeln aufgenommen wurden. Dazu werden wie beschrieben die auflösungsgesteigerten Bilder (vorzugsweise im Fourierraum) mit einem Korrekturbild entfaltet. Das Korrekturbild hat die selbe Pixelzahl wie das auflösungsgesteigerte Bild, wobei alle Pixel den Wert Null aufweisen mit Ausnahme eines quadratischen Bildbereiches mit n*n Pixeln mit den Werten $1/n^2$. Dieses Korrekturbild wird in den Fourierraum transformiert und der Betrag dieser komplexen Funktion gebildet. Das auflösungsgesteigerte Bild wird ebenfalls in den Fourierraum transformiert und durch den Betrag der Fouriertransformierten (FFT) der Korrekturfunktion geteilt. Das so berechnete Bild wird in den Ortsraum zurücktransformiert und ist jetzt einem Bild mit n-facher Auflösung mathematisch fast vollständig äquivalent.

Beispielsweise wird für die Verdoppelung der Auflösung ein Bild mit einer Auflösung von 1000 x 1000 Pixel aufgenommen. Als zweites wird das Bild auf dem Bildaufnahmemodul für eine weitere Aufnahme erst einen halben Pixel horizontal, dann einen halben Pixel vertikal, danach einen halben Pixel horizontal zurück und danach einen halben Pixel vertikal zurück verschoben. Wenn man die vier so erhaltenen Bilder in einander schachtelt erhält man ein Bild mit doppelter Auflösung in beiden Richtungen. Allerdings sind die Pixel mit denen das Gesamtbild mit doppelter Auflösung aufgenommen wurde, doppelt so groß als sie sein müssten, so dass das Bild zwar die doppelte Auflösung hat, aber die Schärfe des Bildes schlechter ist, als die eines Bildes einer Kamera mit 4 Megapixel. Dieses Problem kann behoben werden, wenn das geschachtelte Bild im Fourierraum korrigiert wird, indem das Bild fouriertransformiert und durch den komplexen Betrag der Fouriertransformation der Verteilung eines Korrekturbildes geteilt wird. Das Korrekturbild ist ein Bild mit 1000 x 1000 Pixel mit dem Wert Null und 2 x 2 Pixel mit dem Wert 1/4. Nach der Rücktransformation des so korrigierten Bildes erhält man ein Bild mit echter doppelter Auflösung in jeder Raumrichtung.

Eine Verdreifachung der Auflösung kann dabei durch Aufnahme von 9 Bildern mit einer Verschiebung von jeweils einem drittel Pixel erreicht werden. Höhere Auflösungserhöhungen machen meist keinen Sinn mehr, da durch die Fourierkorrektur auch das im Bild enthaltene Rauschen verstärkt wird.

Der wesentliche Unterschied zum Stand der Technik kann nun so beschrieben werden, das die Bilder nicht aktiv gegeneinander verschoben werden, sondern es wird die Sakadenbewegung der Augen für die Bildverschiebung im Subpixelbereich ausgenutzt. Das bedeutet, das man statistische Bildverschiebungen durch die Sakadenbewegung aufzeichnet. Man muss dann nicht mehr die Bildverschiebungen mit Subpixelgenauigkeit einstellen, sondern nur noch die Bildverschiebung durch eine Korrelation mit Subpixelgenauigkeit berechnen.

Die Korrelationsauflösung kann erheblich vergrößert werden, wenn der Peak im Korrelationsbild, der meist mehrere Pixel in jede Richtung breit ist, durch eine Funktion, wie beispielsweise eine Parabel mit variabler Breite und Scheitelpunktslage angenähert wird. So kann die Lage des Maximums der Korrelationsfunktion und damit auch der relativen Bildverschiebung gegen das erste Bild mit Subpixelgenauigkeit bestimmt werden und genau das ermöglicht die Anwendung der eben beschriebenen Algorithmen zur Auflösungserhöhung.

Im einzelnen wird dazu von der Auswerteeinheit die Verschiebung der vom Bildaufnahmemodul gelieferten Bilder im Subpixelbereich ermittelt, indem das hellste Pixel des Korrelationsbildes und seine beiden nächsten Nachbarn in x-Richtung bestimmt und das Gleichungssystem

$$y_1 = A \cdot (x_1 - x0)^2 + B$$

$$y_2 = A \cdot (x_2 - x0)^2 + B$$

$$y_3 = A \cdot (x_3 - x0)^2 + B$$

gelöst, der Wert der ermittelten Verschiebung in den ganzzahligen Teil und den Subpixelrest zerlegt und die Bilder so verschoben werden, dass der ganze Anteil der ermittelten Verschiebung kompensiert wird, wobei sich dieser Ablauf für die y-Richtung noch einmal wiederholt.

Zur Verdoppelung der Auflösung werden von der Auswerteeinheit die vom Bildaufnahmemodul gelieferten und um jeweils den ganzzahligen Teil in x- und y-Richtung verschoben aufgenommenen Bilder, in ein doppelt so großes Bildfeld pixelweise so einschreiben, dass die Werte mit geradzahligem Pixelindex das Teilbild 1 und die mit ungeradzahligem Pixelindex das Teilbild 2 bilden und für jedes Teilbild eine auf Null gesetzte Zählvariable zugeordnet ist, wobei ein Bild dessen Subpixelrest der Verschiebung zum Referenzbild zwischen 0.25 und 0.75 liegt zum Teilbild 1 addiert und die entsprechende Zählvariabel um 1 erhöht wird, ein Bild dessen Subpixelrest der Verschiebung zum Referenzbild zwischen 0 und 0,25 oder 0,75 und 1 liegt zum Teilbild 2 addiert und die entsprechende Zählvariable um 1 erhöht wird, sich diese Prozedur für alle Bilder und äquivalent für die y-Richtung wiederholt und durch Division der Teilbilder durch die zugehörigen Zählervariablen ein Bild mit doppelter Auflösung entsteht.

Im Einzelnen müssen dazu die Verschiebungen der Bilder gegeneinander mit Subpixelgenauigkeit bestimmt werden. Dazu wird als erstes ein Masterteilbild ausgewählt, gegen das alle Einzelbilder kreuzkorreliert werden.

Als nächster Schritt werden an den hellsten Pixel und seine nächsten beiden Nachbarn in x- und in y-Richtung der Korrelationsfunktion zwei Parabeln angepasst, indem das bereits genannte Gleichungssystem mit 3 Gleichungen und drei unbekannten Parametern für die beiden Parabeln gelöst wird. Die Lage des Intensitätsmaximums gibt die Verschiebung der Bilder zum Masterbild an. Für die Berechnung des auflösungsgesteigerten Bildes wird ein neues Bild mit doppelter Auflösung in beiden Raumrichtungen angelegt, wobei jetzt 2 x 2 Pixel einem Pixel des Ursprungsbildes zugeordnet werden. Die Bilder der Sequenz werden um den ganzen Anteil der Pixelverschiebung zurückgeschoben. Damit sind nun die Bilder in jedem Fall um weniger als ein Pixel gegen das Masterbild verschoben. Als nächstes werden die Teilbilder zu dem Bild mit doppelter Auflösung addiert. Zu welchem der 4 Unterbilder die Teilbilder addiert werden, hängt von der Größe der Subpixelverschiebung ab. Die Subpixelverschiebung wird in Intervalle eingeteilt und jedes Intervall einem Unterbild zugeordnet. Die so addierten Unterbilder werden durch die jeweilige Anzahl an Bildern geteilt, um sie arithmetisch zu mitteln.

Durch diese Prozessschritte erhält man ein Bild mit der doppelten Auflösung. Dieses Bild unterscheidet sich aber von einem Bild, das mit einem doppelt so aufgelösten Kamerachip aufgenommen wurde. Da die Kamerapixel ja eine Größe haben, die der einfachen Auflösung entspricht, ist es zwar möglich durch mehrfaches sampeln mit Subpixelverschiebungen mehr Datenpunkte zu akquirieren. Durch die relativ zu großen Pixel der Kamera werden aber hohe Ortsfrequenzen im auflösungsgesteigerten Bild unterdrückt bzw. gedämpft. Dieser Effekt kann aber im Fourierraum durch einen entsprechenden inversen Filter kompensiert werden. Durch diesen Filter werden die hohen Ortsfrequenzen im Bild verstärkt. Damit erscheint das Bild schärfer aber auch verrauschter.

Der Algorithmus wird für eine Verdreifachung der Auflösung leicht abgeändert. In diesem Fall werden in jeder Richtung drei Teilbilder mit Subpixelverschiebungen in den Intervallen (1/6 ... 3/6, 3/6 ... 5/6, 5/6 ... 1/6) angelegt und die Bilder nach der Größe der Subpixelverschiebung in diese Intervallklassen einsortiert.

Bei den bisher beschriebenen Verfahrensschritten ist es besonders wichtig Bilder zur weiteren Verarbeitung auszuwählen, die möglichst wenig oder keine Fehler beinhalten.

Die Größe m der Teilbilder muss so gewählt werden, dass sie erstens deutlich größer als die maximal zu erwartende Verschiebung der Bilder sind und zweitens eine Mindestgröße nicht unterschreitet, damit sich die durch das Pixelrauschen der Kamerabilder verursachten Fehler in der Korrelationsfunktion über die Teilbilder herausmitteln. Aus einer ungünstigen Wahl der Teilbilder können zusätzliche Fehler resultieren.

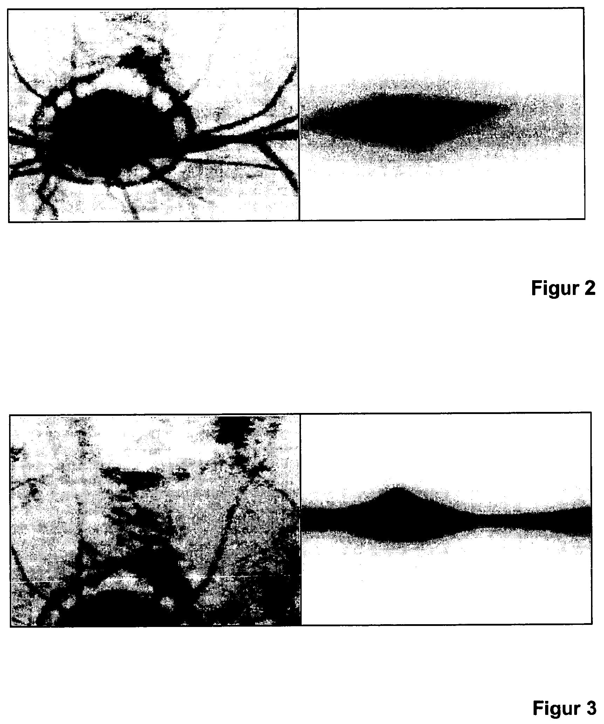

Treten beispielsweise starke Helligkeitsschwankungen an den Rändern der für die Berechnung der Korrelationsfunktion ausgewählten Teilbilder auf, so können Artefakte in der Kreuzkorrelationsfunktion verursacht werden. **Figur 2** zeigt dazu einen ungünstig gewähltes Teilbild und das Ergebnis der resultierenden Korrelationsfunktion. Der hellste Pixel ist hierbei nicht zu ermitteln. Diese Artefakte können jedoch durch die Wahl eines geeigneten Teilbildes vermieden werden. Dazu zeigt **Figur 3** ein geeignetes Teilbild mit dem Ergebnis der resultierenden Korrelationsfunktion. Aus diesem Grund ist für alle weiteren Auswertungen darauf zu achten, dass für die Teilbilder möglichst günstige Teilbilder ausgeschnitten werden.

Insbesondere sollte dafür ein Bereich aus dem Fundusbild ausgewählt werden, in dem klar in x- und y-Richtung strukturierte Bildelemente, wie Adern, Ablagerungen o. ä. vorkommen. Außerdem ist darauf zu achten, dass die Teilbilder entweder relativ gleichmäßig ausgeleuchtet sind oder dass die Helligkeit am Rand des Bildbereiches nicht stark variieren. Aus diesem Grund wurden bei den Angiographieaufnahmen, bei denen die Adern als helle Strukturen zu erkennen sind, ein Teilbild um die helle Papille des Auges ausgewählt.

Aus der Korrelationsfunktion zwischen Masterbild und einem Sequenzbild soll die für die Entwackelung wichtige Verschiebung mit Subpixelgenauigkeit bestimmt werden. Die Genauigkeit ist dabei um so höher, je schmaler und heller der Korrelationspeak ist. Deshalb muss ein möglichst wenig interlacetes und unscharfes Bild ausgewählt werden. Für die Berechnung des gemittelten und/oder auflösungsgesteigerten Bildes ist es auch sehr wichtig, unscharfe oder interlacete Bilder auszusortieren, da diese das Endbild der Algorithmen verschlechtern (verwaschen).

Ziel ist es deshalb einen Algorithmus für das Aussortieren der unscharfen bzw. interlaceten Bilder zu verwenden, der die ungeeigneten Bilder möglichst automatisch und schnell erkennt. Daher wurde versucht für die Ableitung der Messgröße für die Stärke der Bildschärfe und die Interlacefehler auf bereits berechnete Größen der Bilder zurückzugreifen.

Von der Auswerteeinheit werden die vom Bildaufnahmemodul gelieferten Bilder auf Interlacefehler untersucht, indem die 2 dimensionale Fouriertransformierte gebildet, der Ort der höchsten Ortsfrequenzen ermittelt (Summe der Pixelwerte der mittlere Zeile bzw. der mittleren Spalte des 2-dimensionalen Fourierbildes) und die beiden Werte dividiert werden. Bilder deren Quotient dieser beiden Größen deutlich von 1 abweicht, beinhalten Interlacefehler und werden nicht weiterverarbeitet.

Bei einer sogenannten Interlaced-Kamera werden 2 Halbbilder mit jeweils allen geraden Zeilen und einen Takt später allen ungeraden Zeilen ausgelesen. Die Zeit zwischen den Auslesevorgängen der beiden Halbbilder beträgt ca. 20ms. In dieser Zeit kann sich das Bild des Fundus auf der Kamera verschieben, was zur Folge hat, dass die beiden Kamerahalbbilder gegeneinander verschoben sein können. Diese Verschiebung der beiden Halbbilder führt zu Bildfehlern bei der Bildmittelung und Auflösungssteigerung.

Da die beiden Halbbilder in beiden Richtungen gegeneinander verschoben sein können, ist es prinzipiell nicht möglich, die Interlace-Fehler rechnerisch zu kompensieren. Um zu verhindern, das Bilder mit diesen Artefakten von den Algorithmen zur Auflösungssteigerung verarbeitet werden und dadurch das Endbild verwaschen, müssen die entsprechenden Bilder sicher aussortiert werden.

Dies kann beispielsweise erfolgen, indem für die Berechnung der Korrelation ein Bildbereich ausgeschnitten und mit den Algorithmen der schnellen FFT in den Frequenzraum transformiert wird. Die Interlacefehler werden durch Bewegungen während der sequenzielle Aufnahme der Halbbilder mit geradem und ungeradem Zeilenindex verursacht. Diese Fehler führen zu typischen "ausgefransten" vertikalen Kanten im Bild. Dadurch werden im Frequenzspektrum der Bildspalten die Frequenzkomponenten mit der höchsten Ortsfrequenz erhöht.

Diese Erhöhung ist aber im Frequenzspektrum der Zeilen nicht zu erkennen. Daher wird vom Bild $(x_i, y_j)$ die 2-dimensionale FFT berechnet.

$$FFT_{Bild}\left(vx_i, vy_j\right)$$

mit

i, j 0 ... 256,
x Zeilenkoordinate und
y Spaltenkoordinate.

Von diesem Bild werden folgende zwei Größen abgeleitet:

$$S_{vx} = \sum_{\forall i} FFT_{Bild}(vx_i, vy_{127}) \qquad S_{vy} = \sum_{\forall j} FFT_{Bild}(vx_{127}, vy_j)$$

Ist das Bild in x- und y- Richtung ähnlich strukturiert und treten keine Interlaceartefakte auf, so sollte der Quotient $S_{vy}/S_{vx}$ nahe 1 liegen. Treten Interlaceartefakte auf, so wird sich der Quotient erhöhen. Für das Aussortieren der interlaceten Bilder hat sich folgende Schwelle als sinnvoll erwiesen:

$$\frac{S_{vy} - S_{vx}/2}{S_{vx}/2} \quad \begin{array}{l} < 4 \text{ Bilder sind nicht interlaced} \\ > 4 \text{ Bilder sind interlaced} \end{array}$$

Diese Berechnung wird für das Masterbild und jedes Einzelbild der Sequenz empfohlen, wenn die Bilder vollautomatisch bearbeitet werden sollen.

Das Aussortieren von unscharfen Bildern ist deutlich schwieriger, da keine absolute Schwelle wie bei den Interlacefehlern definiert werden kann. Eine relative Wertung der Schärfe der einzelnen Bilder der Sequenz kann wie folgt realisiert werden. Es wird die Korrelationsfunktion aus Masterbild und Einzelbild berechnet. Der hellste Pixel dieser Funktion und seine nächsten beiden Nachbarn in x-Richtung werden durch eine Parabel mit der Gleichung

$$y = A \cdot (x - x0)^2 + B$$

und den drei zu bestimmenden Parametern A, x0 und B angenähert. Die Größe x0 ist die gesuchte Verschiebung der beiden Bilder mit Subpixelgenauigkeit. Die Größe A ist ein Maß für die Breite der Kreuzkorrelationsfunktion zwischen Master- und Einzelbild und damit bei festem Masterbild ein relatives Maß für die Schärfe des Einzelbildes. Diese Rechnung wird für die y-Richtung wiederholt. Überschreitet einer der beiden Parameter $A_x, A_y$ eine bestimmte angepasste Schwelle, so wird das Bild als zu unscharf aussortiert. Die Berechnung der Größen $A_x, A_y$ erfordert keine wesentlich zusätzliche Rechenzeit, da sie bei der Bestimmung von x0 als Zusatzergebnis anfällt.

Um das schärfste Bild aus einer Sequenz als Masterbild auszuwählen, kann der beschriebene Algorithmus für die Autokorrelationsfunktion der Einzelbilder durchgeführt werden. Das schärfste Bild ist das Bild mit den kleinsten A-Parametern. Im Gegensatz zur Bestimmung der Schärfe der Einzelbilder bei der Korrelation gegen das Masterbild verursacht diese Rechnung aber erheblichen rechnerischen Mehraufwand.

Mit der erfindungsgemäßen Einrichtung und dem Verfahren zur Beobachtung, Dokumentation und/oder Diagnose des Augenhintergrundes wird eine Lösung zur Verfügung gestellt, die eine sequentielle, multispektrale Beleuchtung der Retina, bei hohen Bildwinkeln ermöglicht und die Lichtbelastung des zu untersuchenden Auges auf ein Minimum reduziert, indem nur mit den Wellenlängen angeregt wird, die auch der Auswertung dienen.

Das multispektrale, sequentielle, auf monochromatischen LEDs basierende Beleuchtungsmodul ermöglicht eine spektral schmal einstellbare und schnell schaltbare Beleuchtung des zu untersuchenden Objektes. Durch Überlagerung sequentieller Bilder, einer monochromen, empfindlichen, hochauflösenden Kamera ist es durch elektronische Nachbearbeitung möglich, sowohl Fluoreszenz-, Monochrom- und Farbaufnahmen aufzuzeichnen als auch eine diagnostische Auswertung, bei geringst möglicher retinaler Strahlenbelastung durchzuführen.

Vorteilhaft ist dabei die Verwendung nur einer einzigen, monochromatischen Digitalkamera. Außer bei Fluoreszenz-Aufnahmen, bei dem ein Filter in den Beobachtungsstrahlengang eingeschwenkt wird, kann dann auf sämtliche mechanisch bewegen Teile wie Filter und Shutter verzichtet werden.

Mit dem erfindungsgemäßen Verfahren wird eine Lösung zur Verfügung gestellt, mit der es möglich ist, die Fliessgeschwindigkeit von Blut in den Adern des Augenhintergrundes zu bestimmen. Die Größe der Fliessgeschwindigkeit des Blutes in Verbindung mit Angaben der $O_2$-Sättigung im Augenhintergrund liefern neben Informationen über die im Gewebe deponierte Sauerstoffmenge. Auf diese Weise können Störungen der Durchblutung und insbesondere im Metabolismus (Stoffwechsel) diagnostiziert werden.

**Patentansprüche**

1. Einrichtung zur Beobachtung, Dokumentation und/oder Diagnose des Augenhintergrundes, insbesondere des Blutflusses in der Retina, bestehend aus einem ophthalmologischen Untersuchungsgerät, einem multispektralen, sequentiellen Beleuchtungsmodul (1), einem Bildaufnahmemodul (4), einem Steuer- und Sicherheitsmodul (5) und einer Auswerteeinheit (7), bei der:

das an das ophthalmologische Untersuchungsgerät angekoppelte Beleuchtungsmodul (1) aus mindestens zwei, in Intensität und Dauer einzeln regelbaren, monochromatisches Licht verschiedener Wellenlängen abstrahlenden Einzellichtquellen (6.1, 6.2) besteht,

das ophthalmologische Untersuchungsgerät so ausgebildet ist, dass das ebenfalls an das ophthalmologische Untersuchungsgerät angekoppelte Bildaufnahmemodul (4) auf die Wellenlängen des vom Beleuchtungsmodul (1) emittierten Lichtes abgestimmt und mit deren Leuchtdauer synchronisiert ist, wobei

das Steuer- und Sicherheitsmodul (5) ausgebildet ist die zeitliche Abfolge, Dauer und Intensität der einzelnen Lichtquellen (6.1, 6.2) des Beleuchtungsmoduls (1) die für das jeweilige Bild erforderlich sind zu steuern und die Lichtbelastung des Auges (3) zu überwachen und

die Auswerteeinheit (7) ausgebildet ist das Steuer- und Sicherheitsmodul (5) zu regeln und die vom Bildaufnahmemodul (4) übermittelten Aufnahmen des Fundus zu bewerten, zu korrigieren, zu verbessern, zu kombinieren, ortsgenau zu überlagern und auszuwerten

charakterisiert dadurch, dass

das ophthalmologische Untersuchungsgerät so ausgebildet ist, dass das vom Beleuchtungsmodul (1) kommende Licht über das zu untersuchende Auge (3) auf dem Bildaufnahmemodul (4) abgebildet wird.

2. Einrichtung nach Anspruch 1, **dadurch gekennzeichnet, dass** das Beleuchtungsmodul (1) zur Abstrahlung monochromatischer Strahlung im transparenten Bereich okulärer Medien von 350 - 900 nm und 1000 -1150 nm ausgebildet ist, wobei die Einzellichtquellen (6.1, 6.2) eine spektrale Halbwertsbreite von unter 40 nm aufweisen.

3. Einrichtung nach den Ansprüchen 1 und 2, **dadurch gekennzeichnet, dass** das multispektrale, sequentielle Beleuchtungsmodul (1) über mindestens eine, räumlich und zeitlich sehr kohärentes Licht abstrahlende Einzellichtquelle (6.1, 6.2) verfügt.

4. Einrichtung nach Anspruch 3, **dadurch gekennzeichnet, dass** die räumlich und zeitlich sehr kohärentes Licht abstrahlende Einzellichtquelle (6.1) des Beleuchtungsmoduls (1) ein vorzugsweise gepulst betriebener Laser ist.

5. Einrichtung nach Anspruch 4, **dadurch gekennzeichnet, dass** zur Reduzierung der Kohärenz von Laserstrahlung zur flächigen Beleuchtung des Augenhintergrundes bei sehr hohen Strahlungsintensitäten ein veränderliches Streufilter oder ein rotierender Spiegel vorhanden ist.

6. Einrichtung nach den Ansprüchen 1 bis 3, **dadurch gekennzeichnet, dass** das Beleuchtungsmodul (1) zur flächigen und hochenergetischen Beleuchtung des Augenhintergrundes über mindestens eine Einzellichtquelle (6.1) in Form einer pulsförmig betriebenen Gasentladungslichtquelle verfügt.

7. Einrichtung nach den Ansprüchen 1 bis 6, **dadurch gekennzeichnet, dass** das Beleuchtungsmodul (1) aus Einzellichtquellen (6.1, 6.2) besteht, deren Ein- und Ausschaltverzögerung unter 1 ms liegt.

8. Einrichtung nach den Ansprüchen 1 bis 7 , **dadurch gekennzeichnet, dass** die Einzellichtquellen zwei- oder auch dreidimensional angeordnet sind.

9. Einrichtung nach den Ansprüchen 1 bis 8, **dadurch gekennzeichnet, dass** zur Kopplung der von den Einzellichtquellen (6.1, 6.2) erzeugten Strahlenbündel Spiegel, dichroitische Spiegel und/oder Gitter vorhanden sind.

10. Einrichtung nach Anspruch 1, **dadurch gekennzeichnet, dass** die Steuer- und Sicherheitsmodul (5) ausgebildet ist Dauer und Intensität der Einzellichtquellen (6.1, 6.2) an die Verteilung der Farbinformation des abzulichtenden Objektes anzupassen.

11. Einrichtung nach den Ansprüchen 1 und 10, **dadurch gekennzeichnet, dass** das Steuer- und Sicherheitsmodul (5) ausgebildet ist das Bildaufnahmemodul (4) synchron zum Beleuchtungsmodul (1) zu steuern, so dass zu jeder Wellenlänge eine, oder vorzugsweise auch mehrere zugehörige Bilder mit optimalem Kontrast realisiert werden.

12. Einrichtung nach Anspruch 1, **dadurch gekennzeichnet, dass** das Bildaufnahmemodul (4) eine empfindliche Monochromkamera enthält, deren Bildwandelchip ein Mikrolinsen-Array vorgeordnet ist.

13. Einrichtung nach Anspruch 1, **dadurch gekennzeichnet, dass** die Auswerteeinheit (7) ausgebildet ist die vom Bildaufnahmemodul (4) gelieferten Bilder hinsichtlich Kontrast, Bildschärfe, Farbfehler, Pixelfehler, Randabfall, Verzeichnungen, örtlichem Versatz u. ä. weiter zu verarbeiten.

**14.** Einrichtung nach den Ansprüchen 1 und 13, **dadurch gekennzeichnet, dass** die Auswerteeinheit (7) ausgebildet ist aus mehreren vom Bildaufnahmemodul (4) gelieferten monochromatischen Bildern ein gemitteltes Bild oder ein farbiges Bild des Fundus zu ermitteln.

**15.** Einrichtung nach den Ansprüchen 1, 13 und 14, **dadurch gekennzeichnet, dass** die Auswerteeinheit (7) ausgebildet ist aus mehreren vom Bildaufnahmemodul (1) gelieferten Einzelbildern ein Bild mit einer n-fach größeren Auflösung in jeder Raumrichtung zu berechnen.

**16.** Einrichtung nach Anspruch 1, **dadurch gekennzeichnet, dass** das ophthalmologische Untersuchungsgerät eine, nach dem mydriatischen oder non-mydriatischen Prinzip arbeitende Funduskamera (2) ist.

**17.** Einrichtung nach Anspruch 1, **dadurch gekennzeichnet, dass** die Auswerteeinheit (7) ausgebildet ist die gewonnenen Daten, in Form von Einzelaufnahmen, kombinierten sowie mittels Software bearbeiteten Einzelaufnahmen sowie Aufnahme von Live-Sequenzen mit mindestens 10 Bildern pro Sekunde, zeitnah und dauerhaft zu speichern.

**18.** Verfahren zur Beobachtung, Dokumentation und/oder Diagnose des Augenhintergrundes, insbesondere des Blutflusses in der Retina, bei dem mit Hilfe eines ophthalmologischen Untersuchungsgerätes das Licht eines multispektralen, sequentiellen Beleuchtungsmoduls (1) über das Auge (3) auf ein Bildaufnahmemodul (4) abgebildet wird, wobei

das an das ophthalmologische Untersuchungsgerät angekoppelte, aus mindestens zwei, in Intensität und Dauer einzeln regelbare Einzellichtquellen (6.1, 6.2) bestehende Beleuchtungsmodul (1), monochromatisches Licht verschiedener Wellenlängen abstrahlt, welches vom ophthalmologischen Untersuchungsgerät über das zu untersuchende Auge (3) auf dem ebenfalls an das ophthalmologische Untersuchungsgerät angekoppelte Bildaufnahmemodul (4), welches auf die Wellenlängen des vom Beleuchtungsmodul emittierten Lichtes abgestimmt und mit deren Leuchtdauer synchronisiert ist, abgebildet wird, wobei

vom Steuer- und Sicherheitsmodul (5) die zeitliche Abfolge, Dauer und Intensität der Einzellichtquellen (6.1, 6.2) des Beleuchtungsmoduls (1), die für die jeweiligen Bilder des Fundus erforderlich sind gesteuert und die Lichtbelastung des zu untersuchenden Auges (3) überwacht und

von der Auswerteeinheit (7) das Ansteuer- und Sicherheitsmodul (5) geregelt und die vom Bildaufnahmemodul (4) übermittelten Bilder des Fundus bewertet, korrigiert, verbessert, kombiniert, ortsgenau überlagert und ausgewertet werden.

**19.** Verfahren nach Anspruch 18, **dadurch gekennzeichnet, dass** die Einzellichtquellen (6.1, 6.2) des Beleuchtungsmoduls (1) eine Ein- und Ausschaltverzögerung von unter 1 ms aufweisen.

**20.** Verfahren nach Anspruch 18, **dadurch gekennzeichnet, dass** das Steuer- und Sicherheitsmodul (4) Dauer und Intensität der Einzellichtquellen (6.1, 6.2) an die Verteilung der Farbinformation des abzulichtenden Objektes angepasst.

**21.** Verfahren nach den Ansprüchen 18 und 20, **dadurch gekennzeichnet, dass** das Steuer- und Sicherheitsmodul (5) das Bildaufnahmemodul (4) synchron zum multispektralen Beleuchtungsmodul (1) steuert, so dass zu jeder Wellenlänge eine, oder vorzugsweise auch mehrere zugehörige Bilder mit optimalem Kontrast realisiert werden.

**22.** Verfahren nach Anspruch 18, **dadurch gekennzeichnet, dass** das Bildaufnahmemodul (4) eine empfindliche Monochromkamera enthält, deren Bildwandelchip ein Mikrolinsen-Array vorgeordnet ist.

**23.** Verfahren nach Anspruch 18, **dadurch gekennzeichnet, dass** die Auswerteeinheit (7) die vom Bildaufnahmemodul (4) gelieferten Bilder hinsichtlich Kontrast, Bildschärfe, Farbfehler, Pixelfehler, Randabfall, Verzeichnungen, örtlichem Versatz u. ä. weiterverarbeitet.

**24.** Verfahren nach den Ansprüchen 18 und 23, **dadurch gekennzeichnet, dass** die Auswerteeinheit (7) aus mehreren vom Bildaufnahmemodul (4) gelieferten monochromatischen Bildern ein gemitteltes Bild oder ein farbiges Bild des Fundus ermittelt.

**25.** Verfahren nach den Ansprüchen 18, 23 und 24, **dadurch gekennzeichnet, dass** von der Auswerteeinheit (7) aus mehreren vom Bildaufnahmemodul (4) gelieferten Einzelbildern ein Bild mit einer n-fach größeren Auflösung in jeder Raumrichtung berechnet wird.

26. Verfahren nach Anspruch 18, **dadurch gekennzeichnet, dass** das gepulst betriebene Beleuchtungsmodul (1) für die Doppelaufnahmen hoher zeitlicher Auflösung bei räumlich und zeitlich sehr kohärenter Beleuchtung so vom Steuer- und Sicherheitsmodul (5) gesteuert wird, dass ein erster Beleuchtungsblitz am Ende einer ersten Belichtungszeit und ein zweiter Beleuchtungsblitz am Anfang der zweiten Belichtungszeit ausgelöst wird, damit als Bildaufnahmemodul (4) eine handelsübliche Digitalkamera Verwendung finden kann.

27. Verfahren nach Anspruch 18, **dadurch gekennzeichnet, dass** die Auswerteeinheit (7) die gewonnenen Daten, in Form von Einzelaufnahmen, kombinierten sowie mittels Software bearbeiteten Einzelaufnahmen sowie Aufnahme von Live-Sequenzen mit mindestens 10 Bildern pro Sekunde, zeitnah und dauerhaft speichert.

**Claims**

1. Apparatus for observing, documenting and/or diagnosing the fundus, more particularly the blood flow in the retina, consisting of an ophthalmological examination device, a multispectral, sequential illumination module (1), an image recording module (4), a control and safety module (5) and an evaluation unit (7), in which:

    the illumination module (1) coupled to the ophthalmological examination device consists of at least two individual light sources (6.1, 6.2) individually regulable in terms of intensity and duration, which emit monochromatic light with different wavelengths,
    the ophthalmological examination device is embodied in such a way that the image recording module (4) likewise coupled to the ophthalmological examination device is tuned to the wavelengths of the light emitted by the illumination module (1) and synchronized with the lighting duration thereof, wherein
    the control and safety module (5) is embodied to control the temporal sequence, duration and intensity of the individual light sources (6.1, 6.2) of the illumination module (1) required for the respective image and monitor the light exposure of the eye (3), and
    the evaluation unit (7) is embodied to regulate the control and safety module (5) and assess, correct, improve, combine, superpose in a spatially accurate manner and evaluate the recordings of the fundus transmitted by the image recording module (4),
    **characterized in that**
    the ophthalmological examination device is embodied in such a way that the light coming from the illumination module (1) is imaged on the image recording module (4) via the eye (3) to be examined.

2. Apparatus according to Claim 1, **characterized in that** the illumination module (1) is embodied to emit monochromatic radiation in the transparent range of ocular media of 350-900 nm and 1000-1150 nm, wherein the individual light sources (6.1, 6.2) have a spectral full width at half maximum of less than 40 nm.

3. Apparatus according to Claims 1 and 2, **characterized in that** the multispectral, sequential illumination module (1) comprises at least one individual light source (6.1, 6.2) emitting very spatially and temporally coherent light.

4. Apparatus according to Claim 3, **characterized in that** the individual light source (6.1) of the illumination module (1) emitting very spatially and temporally coherent light is a laser preferably operated in pulsed operation.

5. Apparatus according to Claim 4, **characterized in that** a changeable scattering filter or a rotating mirror is present for reducing the coherence of laser radiation for the two-dimensional illumination of the fundus in the case of very high radiation intensities.

6. Apparatus according to Claims 1 to 3, **characterized in that** the illumination module (1) comprises at least one individual light source (6.1) in the form of a gas discharge light source operated in a pulse-shaped manner for the purposes of two-dimensional and high-energy illumination of the fundus.

7. Apparatus according to Claims 1 to 6, **characterized in that** the illumination module (1) consists of individual light sources (6.1, 6.2), the on and off delay of which is less than 1 ms.

8. Apparatus according to Claims 1 to 7, **characterized in that** the individual light sources are arranged in a two-dimensional or else three-dimensional fashion.

9. Apparatus according to Claims 1 to 8, **characterized in that** mirrors, dichroic mirrors and/or gratings are present for coupling the beams generated by the individual light sources (6.1, 6.2).

10. Apparatus according to Claim 1, **characterized in that** the control and safety module (5) is embodied to adapt the duration and intensity of the individual light sources (6.1, 6.2) to the distribution of the colour information of the object to be photographed.

11. Apparatus according to Claims 1 and 10, **characterized in that** the control and safety module (5) is embodied to control the image recording module (4) synchronously with the illumination module (1) such that one, or else preferably more, associated image(s) with an ideal contrast are realized for each wavelength.

12. Apparatus according to Claim 1, **characterized in that** the image recording module (4) contains a sensitive monochrome camera, with a microlens array being arranged in front of the image transducer chip of said camera.

13. Apparatus according to Claim 1, **characterized in that** the evaluation unit (7) is embodied to further process the images supplied by the image recording module (4) in view of contrast, image sharpness, colour aberrations, pixel errors, edge drop, distortions, spatial offset and the like.

14. Apparatus according to Claims 1 and 13, **characterized in that** the evaluation unit (7) is embodied to establish an averaged image or a colour image of the fundus from a plurality of monochromatic images supplied by the image recording module (4).

15. Apparatus according to Claims 1, 13 and 14, **characterized in that** the evaluation unit (7) is established to calculate an image with an n-times greater resolution in each spatial direction from a plurality of individual images supplied by the image recording module (1).

16. Apparatus according to Claim 1, **characterized in that** the ophthalmological examination device is a fundus camera (2) operating according to the mydriatic or non-mydriatic principle.

17. Apparatus according to Claim 1, **characterized in that** the evaluation unit (7) is embodied to store the obtained data, in the form of individual recordings, combined individual recordings and individual recordings processed by means of software, as well as recordings from live sequences with at least 10 frames per second, in a permanent and timely manner.

18. Method for observing, documenting and/or diagnosing the fundus, more particularly the blood flow in the retina, in which the light from a multispectral, sequential illumination module (1) is imaged on an image recording module (4) via the eye (3) with the aid of an ophthalmological examination device, wherein
the illumination module (1) coupled to the ophthalmological examination device consisting of at least two individual light sources (6.1, 6.2) individually regulable in terms of intensity and duration emits monochromatic light with different wavelengths, which is imaged by the ophthalmological examination device via the eye (3) to be examined on the image recording module (4) which is likewise coupled to the ophthalmological examination device and which is tuned to the wavelengths of the light emitted by the illumination module and synchronized with the lighting duration thereof, wherein
the control and safety module (5) controls the temporal sequence, duration and intensity of the individual light sources (6.1, 6.2) of the illumination module (1), which are required for the respective images of the fundus, and monitors the light exposure of the eye (3) to be examined,
and
the control and safety module (5) is regulated and the images of the fundus transmitted by the image recording module (4) are assessed, corrected, improved, combined, superposed in a spatially accurate manner and evaluated by the evaluation unit (7).

19. Method according to Claim 18, **characterized in that** the individual light sources (6.1, 6.2) of the illumination module (1) have an on and off delay of less than 1 ms.

20. Method according to Claim 18, **characterized in that** the control and safety module (4) adapts duration and intensity of the individual light sources (6.1, 6.2) to the distribution of the colour information of the object to be photographed.

21. Method according to Claims 18 and 20, **characterized in that** the control and safety module (5) controls the image recording module (4) synchronously with the multispectral illumination module (1) such that one, or else preferably more, associated image(s) with an ideal contrast are realized for each wavelength.

**22.** Method according to Claim 18, **characterized in that** the image recording module (4) contains a sensitive monochrome camera, with a microlens array being arranged in front of the image transducer chip of said camera.

**23.** Method according to Claim 18, **characterized in that** the evaluation unit (7) further processes the images supplied by the image recording module (4) in view of contrast, image sharpness, colour aberrations, pixel errors, edge drop, distortions, spatial offset and the like.

**24.** Method according to Claims 18 and 23, **characterized in that** the evaluation unit (7) establishes an averaged image or a colour image of the fundus from a plurality of monochromatic images supplied by the image recording module (4).

**25.** Method according to Claims 18, 23 and 24, **characterized in that** an image with an n-times greater resolution in each spatial direction is calculated by the evaluation unit (7) from a plurality of individual images supplied by the image recording module (4).

**26.** Method according to Claim 18, **characterized in that** the control and safety module (5) controls the illumination module (1) operated under pulsed operation for the double recordings with the high temporal resolution in the case of very spatially and temporally coherent illumination in such a way that a first illumination flash is triggered at the end of a first exposure time and a second illumination flash is triggered at the start of the second exposure time, so that a commercially available digital camera can be used as an image recording module (4).

**27.** Method according to Claim 18, **characterized in that** the evaluation unit (7) stores the obtained data, in the form of individual recordings, combined individual recordings and individual recordings processed by means of software, as well as recordings from live sequences with at least 10 frames per second, in a permanent and timely manner.

## Revendications

**1.** Dispositif destiné à examiner, documenter et/ou diagnostiquer le fond de l'oeil, notamment le flux sanguin dans la rétine, constitué d'un appareil d'examen ophtalmologique, d'un module d'éclairage séquentiel multi-spectral (1), d'un module d'acquisition d'image (4), d'un module de commande et de sécurité (5) et d'une unité d'évaluation (7), dans lequel :

le module d'éclairage (1) couplé à l'appareil d'examen ophtalmologique est constitué d'au moins deux sources lumineuses individuelles (6.1, 6.2) réglables en intensité et en durée rayonnant une lumière monochromatique ayant des longueurs d'onde différentes,
l'appareil d'examen ophtalmologique est conçu de manière à ce que le module d'acquisition d'image (4) également couplé à l'appareil d'examen ophtalmologique soit accordé aux longueurs d'onde de la lumière émise par le module d'éclairage (1) et soit synchronisé avec la durée d'éclairage de cette dernière, dans lequel
le module de commande et de sécurité (5) est conçu pour commander la séquence temporelle, la durée et l'intensité des sources lumineuses individuelles (6.1, 6.2) du module d'éclairage (1) qui sont nécessaires pour l'image respective et pour surveiller la sollicitation lumineuse imposée à l'oeil (3), et
l'unité d'évaluation (7) est conçue pour piloter le module de commande et de sécurité (5) et pour évaluer, corriger, améliorer, combiner, superposer de manière spatialement exacte, et exploiter les acquisitions du fond de l'oeil transmises par le module d'acquisition d'image (4),
**caractérisé en ce que** l'appareil d'examen ophtalmologique est conçu de manière à ce qu'une image de la lumière provenant du module d'éclairage (1) par l'intermédiaire de l'oeil à examiner (3) soit formée sur le module d'acquisition d'image (4).

**2.** Dispositif selon la revendication 1, **caractérisé en ce que** le module d'éclairage (1) est conçu pour émettre un rayonnement monochromatique dans la région transparente de milieux oculaires de 350-900 nm et de 1000-1150 nm, dans lequel les sources lumineuses individuelles (6.1, 6.2) présentent une largeur spectrale à mi-hauteur inférieure à 40 nm.

**3.** Dispositif selon les revendications 1 et 2, **caractérisé en ce que** le module d'éclairage séquentiel multispectral (1) possède au moins une source lumineuse individuelle (6.1, 6.2) émettant une lumière spatialement et temporellement très cohérente.

**4.** Dispositif selon la revendication 3, **caractérisé en ce que** la source lumineuse individuelle (6.1) émettant une lumière spatialement et temporellement très cohérente du module d'éclairage (1) est de préférence un laser attaqué de manière pulsée.

**5.** Dispositif selon la revendication 4, **caractérisé en ce que**, pour réduire la cohérence du rayonnement laser afin d'éclairer superficiellement le fond de l'oeil pour des intensités de rayonnement très élevées, un filtre diffusant modifiable ou un miroir tournant est présent.

**6.** Dispositif selon les revendications 1 à 3, **caractérisé en ce que** le module d'éclairage (1) possède une source lumineuse individuelle (6.1) sous la forme d'une source lumineuse à décharge gazeuse attaquée de manière pulsée pour éclairer de manière superficielle et à haute énergie le fond de l'oeil.

**7.** Dispositif selon les revendications 1 à 6, **caractérisé en ce que** le module d'éclairage (1) est constitué de sources lumineuses individuelles (6.1, 6.2) dont le retard d'activation et de désactivation est inférieur à 1 ms.

**8.** Dispositif selon les revendications 1 à 7, **caractérisé en ce que** les sources lumineuses individuelles sont disposées de manière bidimensionnelle ou également tridimensionnelle.

**9.** Dispositif selon les revendications 1 à 8, **caractérisé en ce que**, pour coupler les faisceaux de rayons générés par les sources lumineuses individuelles (6.1, 6.2), des miroirs, des miroirs dichroïques et/ou des réseaux de diffraction sont présents.

**10.** Dispositif selon la revendication 1, **caractérisé en ce que** le module de commande et de sécurité (5) est conçu pour adapter la durée et l'intensité des sources lumineuses individuelles (6.1, 6.2) à la distribution des informations de couleurs de l'objet à photographier.

**11.** Dispositif selon les revendications 1 et 10, **caractérisé en ce que** le module de commande et de sécurité (5) est conçu pour commander le module d'acquisition d'image (4) en synchronisme avec le module d'éclairage (1) de manière à réaliser, pour chaque longueur d'onde, une image correspondante, ou de préférence également, plusieurs images correspondantes avec un contraste optimal.

**12.** Dispositif selon la revendication 1, **caractérisé en ce que** le module d'acquisition d'image (4) comporte une caméra monochromatique sensible dont la puce de conversion d'image est précédée d'un réseau de microlentilles.

**13.** Dispositif selon la revendication 1, **caractérisé en ce que** l'unité d'évaluation (7) est conçue pour traiter les images délivrées par le module d'acquisition d'image (4) en ce qui concerne le contraste, la netteté, les aberrations de couleur, les défauts de pixels, la décroissance de niveau sur les bords, les déformations d'image, un décalage spatial, ou autres.

**14.** Dispositif selon les revendications 1 et 13, **caractérisé en ce que** l'unité d'évaluation (7) est conçue pour déterminer, à partir de plusieurs images monochromatiques délivrées par le module d'acquisition d'image (4), une image moyennée ou une image en couleur du fond de l'oeil.

**15.** Dispositif selon les revendications 1, 13 et 14, **caractérisé en ce que** l'unité d'évaluation (7) est conçue pour calculer, à partir de plusieurs images individuelles délivrées par le module d'acquisition d'image (1), une image ayant une résolution n fois supérieure dans chaque direction spatiale.

**16.** Dispositif selon la revendication 1, **caractérisé en ce que** l'appareil d'examen ophtalmologique est une caméra de fond de l'oeil (2) fonctionnant selon le principe mydriatique ou non mydriatique.

**17.** Dispositif selon la revendication 1, **caractérisé en ce que** l'unité d'évaluation (7) est conçue pour stocker en temps utile et de manière durable les données acquises, sous la forme d'acquisitions individuelles, d'acquisitions individuelles combinées et traitées par des logiciels, ainsi que d'acquisitions de séquences temps réel comportant au moins 10 images par seconde.

**18.** Procédé destiné à examiner, documenter et/ou diagnostiquer le fond de l'oeil, notamment le flux sanguin dans la rétine, dans lequel, au moyen d'un appareil d'examen ophtalmologique, une image de la lumière d'un module d'éclairage séquentiel multispectral (1) est formée par l'intermédiaire de l'oeil (3) sur un module d'acquisition d'image (4), dans lequel :

le module d'éclairage (1) couplé à l'appareil d'examen ophtalmologique, constitué d'au moins deux sources lumineuses individuelles (6.1, 6.2) réglables en intensité et en durée, émet une lumière monochromatique ayant des longueurs d'onde différentes, dont l'image est formée par l'appareil d'examen ophtalmologique par l'intermédiaire de l'oeil (3) à examiner sur le module d'acquisition d'image (4) également couplé à l'appareil d'examen ophtalmologique, lequel module d'acquisition d'image est accordé aux longueurs d'onde de la lumière émise par le module d'éclairage et est synchronisé avec la durée d'éclairage de cette dernière, dans lequel

le module de commande et de sécurité (5) commande la séquence temporelle, la durée et l'intensité des sources lumineuses individuelles (6.1, 6.2) du module d'éclairage (1), qui sont nécessaires pour l'image respective du fond de l'oeil, et surveille la sollicitation lumineuse imposée à l'oeil (3) à examiner, et

le module de commande et de sécurité (5) est piloté par l'unité d'évaluation (7) et les images du fond de l'oeil transmises par le module d'acquisition d'image (4) sont évaluées, corrigées, améliorées, combinées, superposées de manière spatialement précise et exploitées.

19. Procédé selon la revendication 18, **caractérisé en ce que** les sources lumineuses individuelles (6.1, 6.2) du module d'éclairage (1) présentent un retard d'activation et de désactivation inférieur à 1 ms.

20. Procédé selon la revendication 18, **caractérisé en ce que** le module de commande et de sécurité (4) adapte l'intensité des sources lumineuses individuelles (6.1, 6.2) à la distribution des informations de couleur de l'objet à photographier.

21. Procédé selon les revendications 18 et 20, **caractérisé en ce que** le module de commande et de sécurité (5) commande le module d'acquisition d'image (4) de manière synchrone avec le module d'éclairage multispectral (1) de manière à réaliser, pour chaque longueur d'onde, une image correspondante, ou de préférence également, plusieurs images correspondantes avec un contraste optimal.

22. Procédé selon la revendication 18, **caractérisé en ce que** le module d'acquisition d'image (4) comporte une caméra monochromatique sensible dont la puce de conversion d'image est précédée d'un réseau de microlentilles.

23. Procédé selon la revendication 18, **caractérisé en ce que** l'unité d'évaluation (7) traite de manière supplémentaire les images délivrées par le module d'acquisition d'image (4) en ce qui concerne le contraste, la netteté, les aberrations de couleur, les défauts de pixels, la décroissance de niveau sur les bords, les déformations d'image, un décalage spatial, ou autres.

24. Procédé selon les revendications 18 et 23, **caractérisé en ce que** l'unité d'évaluation (7) détermine, à partir de plusieurs images monochromatiques délivrées par le module d'acquisition d'image (4), une image moyennée ou une image en couleur du fond de l'oeil.

25. Procédé selon les revendications 18, 23 et 24, **caractérisé en ce que** l'unité d'évaluation (7) calcule, à partir de plusieurs images individuelles délivrées par le module d'acquisition d'image (4), une image ayant une résolution n fois supérieure dans chaque direction spatiale.

26. Procédé selon la revendication 18, **caractérisé en ce que** le module d'éclairage (1) attaqué de manière pulsée est commandé par le module de commande et de sécurité (5) pour effectuer des acquisitions doubles ayant une résolution temporelle plus élevée avec un éclairage spatialement et temporellement très cohérent de manière à ce qu'un premier éclair d'éclairage soit déclenché à la fin d'un premier temps d'éclairage et qu'un second éclair d'éclairage soit déclenché au début du second temps d'éclairage, de telle sorte qu'un appareil photo numérique du commerce peut être utilisé en tant que module d'acquisition d'image (4).

27. Procédé selon la revendication 18, **caractérisé en ce que** l'unité d'évaluation (7) stocke en temps utile et de manière durable les données acquises, sous la forme d'acquisitions individuelles, d'acquisitions individuelles combinées et traitées par des logiciels, ainsi que d'acquisitions provenant de séquences temps réel comportant au moins 10 images par seconde.

**Figur 2**

**Figur 3**

Figur 4

**Figur 5**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 2512427 A1
- US 20040075812 A
- EP 1114608 B1
- US 5997141 A
- US 3760174 A
- US 5926283 A1
- US 5982497 A1

- DE 3041178 C2
- US 5983120 A1
- EP 1084674 B1
- EP 0801534 B1
- WO 9705538 A1
- US 6549801 B1
- JP 10314118 A